# EUROPEAN PATENT APPLICATION

(11) **EP 4 796 177 A2**
(43) Date of publication of application: **26.08.2026**
(21) Application number: 26176063.1
(22) Date of filing: 11.10.2024
(51) Int. Cl.: A61P 21/00

(54) **COMPOSITIONS COMPRISING A PROBIOTIC STRAIN FOR IMPROVING MUSCLE STRENGTH**

(30) Priority: 11.10.2023 EP 23202932
(62) Document number(s) of the earlier application(s) in accordance with Art. 76 EPC: 24790348.7 / 4 791 224
(71) Applicant: Société des Produits Nestlé S.A., 1800 Vevey (CH)
(72) Inventor: HORCAJADA, Marie Noëlle, 01170 Echenevex (FR); BONNET, Nicolas, 74160 Beaumont (FR); STUELSATZ, Pascal, 1023 Crissier (CH); DESGEORGES, Thibaut, 1004 Lausanne (CH); GARCIA-GARCERA, Marc, 1509 Vucherens (CH); EGLI, Delphine, 1806 Saint-Légier-La Chiésaz (CH)
(74) Representative: Loiseau, François Michel

(57) **Abstract**

The invention provides a nutritional composition comprising pre-conditioned *L. reuteri* as a probiotic strain. The nutritional composition can be particularly enriched in vitamins and minerals, and is useful for improving the muscle condition of a subject, particularly the muscle strength in an infant, a young child or a child.

## Description

### Field of the invention

This invention relates to nutritional compositions comprising pre-conditioned *L. reuteri* as a probiotic strain, in particular in combinations with micronutrients, especially vitamins and minerals in specific levels for vitamin B3, vitamin C, vitamin D, vitamin E, vitamin K, vitamin B7, vitamin B5, vitamin B9, magnesium, manganese and selenium. The lipids of the nutritional compositions comprise advantageously fatty acid triacylglycerols, said fatty acid triacylglycerols comprising stearic, palmitic and myristic esters, wherein the sum of the amount of stearic, palmitic and myristic esters at the sn-1(3) position in low amounts. The invention also relates the nutritional compositions for use in improving, increasing, treating, stimulating, or promoting the muscle condition in a subject, in particular increasing muscle strength in infants, young children, or children.

### Background of the invention

Skeletal muscle plays essential roles in health maintenance across lifespan. Its main functions in the body are to regulate whole body metabolism, to store amino acids, to produce heat and to generate force to allow movement. Skeletal muscle has a complex architecture. It is composed of anatomical structures called fascicle that are surrounded by connective tissue. Then, all fascicles are composed of myofibers, which are the muscle cells. Those myofibers are syncytia containing multiple nuclei due to the fusion of muscle stem cells. Myofibers contain the contractile apparatus, the myofibril, that is responsible of force generation via the interaction of fibrillar protein, actin and myosin. The interaction between actin and myosin required energy through the degradation of ATP and is controlled by the central nervous system.

Muscle strength is an important aspect of physical fitness and health status, and a decrease of muscle strength may cause significant functional limitations. Therefore, muscle strength is an important outcome. Muscle in healthy children should serve as a reference for children and adolescents with acute and chronic conditions when muscle strength is a measure for diagnostic purposes, follow- up, or to assess the efficacy of therapy. Grip strength was proven to strongly correlate with total muscle strength (Wind et al.; Is grip strength a predictor for total muscle strength in healthy children, adolescents, and young adults? Eur J Pediatr (2010) 169:281-287). Studies have found the association between grip strength and back strength and quadriceps strength in healthy females. A significant correlation coefficient between grip strength and back strength of r=0.501 and between grip strength and quadriceps strength of r=0.536 was reported. Additionally, studies reported a significant correlation between grip strength and arm circumference and arm strength, and other studies reported a significant correlation between grip strength and jumping strength. Also, several studies reported a significant association between grip strength and physical fitness or health status.

Mckirdy et al. have shown that handgrip strength is predictive of fat free mass and might be used as a complimentary method to screen for nutritional risk and the need for further review and dietetic intervention on admission to hospital. (Handgrip strength as a surrogate marker of lean mass and risk of malnutrition in paediatric patients. Clin Nutr. 2021 Sep; 40(9): 5189-5195. doi: 10.1016/j.cInu.2021.08.005: 10.1016/j.clnu.2021.08.005).

Muscle tissue is extremely plastic and has the incredible capacity to adapt to needs. It can be hypertrophic in response to strength exercise for example, or it can also be atrophic if not used or under pathological condition. Across lifespan and particularly during early life, muscle tissue expends a lot during the childhood. There are two main mechanisms that allows skeletal muscle growth during development: one mechanism involving the muscle stem cells and one mechanism through protein synthesis, both will increase the size of the myofibers (Verdijk, L. B., et al. (2014). "Satellite cells in human skeletal muscle; from birth to old age." Age (Dordr) 36(2): 545-547. Evangelista, T., et al. (2022). "Comprehensive morphometric assessment of deltoid muscle development in children: A cross-sectional study." EBioMedicine 86: 104367.). The 2 mechanisms occur in parallel. Overall, three main factors are recognized to contribute to an optimal muscle growth during childhood: growth hormones and sex steroids, physical activity, and nutrition (Orsso, C. E., et al. (2019). "Low muscle mass and strength in pediatrics patients: Why should we care?" Clin Nutr 38(5): 2002-2015). The most described impact of nutrition is through the protein intake that has been identified in many studies as a requirement for a proper muscle growth. According to the World Health Organization, the average requirement of protein in healthy children ranges from 1.12 g/kg/day to 0.75 g/kg/day at 6 months and 10 years, respectively (Orsso et al., 2019). Besides protein, other nutrients could have a positive impact on muscle growth such as vitamins or minerals.

It has been found that folate plays an important role not only in the development of muscle cells in vivo and in vitro. Vitamin B9 and is important for health, growth, and development. As a precursor of various cofactors, folate is required for one-carbon donors in the synthesis of DNA bases and other essential biomolecules. A lack of dietary folate can lead to folate deficiency and can therefore result in several health problems, including muscle weakness.

Folate deficiency affects muscle cell development. Several studies have implied that folate might exert a positive effect on skeletal muscle development. However, the precise effects of folate in skeletal muscle development are still poorly understood. A recent study reveals that serum folate levels are significantly correlated to reduction of leg and grip strength in older people. Reduced dietary intake of micronutrients including folate has major impact on muscle health as shown by decreased force generating capacity and fatigue resistance as well as impaired physical activity, without an effect on muscle mass in aged mice (Hwang et al.; Arch. Pharm. Res. (2019) 42:319-325).

A study reported the association between serum vitamin levels and muscle strength and gait measures in patients >65 years old with diabetes mellitus in a primary care setting (Wee, Nutrition Journal (2016) 15:89). Vitamin B12 deficiency (<150 pmol/l) was present in 43 % of patients, folate deficiency (<13.5 mmol/l) in 20 %, hyperhomocysteinaemia (≥15.0 µmol/l) in 52 % and vitamin D deficiency (<49.9 nmol/l) in 25 %. Levels of vitamin D, vitamin B12 and homocysteine did not significantly predict muscle strength in regression analyses. Folate (B = 0.010, P < 0.01) and gender (B = 0.356, P < 0.001) predicted average grip strength corrected for BMI (F(2,53) = 17.74, P < 0.001, R2 = 0.40). Folate (B = 0.011, P < 0.05) and gender (B = 0.367, P < 0.001) also predicted average leg quadriceps strength corrected for BMI. However, the influence of folate on muscle strength in early life remains unexplored.

More recently, microbiota has been described to also have an impact on muscle growth. Indeed, studies have shown that nutrition can interfere with gut microbiota which can subsequently impacts on muscle growth in infant (Qi, R., et al. (2021). "The intestinal microbiota contributes to the growth and physiological state of muscle tissue in piglets." Sci Rep 11(1): 11237. Blanton, L. V., et al. (2016). "Gut bacteria that prevent growth impairments transmitted by microbiota from malnourished children." Science 351(6275)).

*Bifidobacteria* are known to be implicated in the enhancement on the muscle mass in infants and skeletal muscle, i.e., the gut-muscle axis (Toda et al.; Heat-Killed Bifidobacterium breve B-3 Enhances Muscle Functions: Possible Involvement of Increases in Muscle Mass and Mitochondrial Biogenesis. Nutrients 2020, 12, 219).

It has been demonstrated that probiotic *Streptococcus thermophilus FP4* and *Bifidobacterium breve BR03* supplementation attenuates the performance and range-of-motion decrements following muscle damaging exercise (Jaeger et al.; Nutrients 2016, 8, 642).

WO2022/191767 A1 describes the preparation of a blend of GOS with pre-conditioned *L. reuteri* and its use to stimulate of osteoblast mineralization.

Fatty acids benefit muscles in animals by improving the lipid profile, and enhancing the antiinflammatory and mitochondrial function. However, more well-designed studies are required to evaluate the dosage and duration of using fatty acids in humans. Intake of omega-3 fatty acids for more than 6 months may be a potential choice for elderlies to prevent or improve sarcopenia as it has positive effects on muscles (Huang et al., Nutrients, 2023, 15, 3613). However, the influence of lipids on muscle strength in early life remains unexplored.

Therefore, there is an interesting opportunity to identify nutrients or compositions that could sustain muscle growth for maintaining muscle health. Such nutrients or compositions may help subjects to develop and maintain a proper muscle growth during infancy and childhood by facilitating maintenance of or increasing muscle strength, muscle function and/or muscle mass.

There remains a need to develop a composition to improve the muscle condition, especially in early life subjects.

There is a need to further improve such compositions which are easy to administer, like during the diet, and which does not have the side effects of a medicament while ensuring similar benefits.

### Summary of the invention

The present inventors have surprisingly found that administering a nutritional composition comprising *L. reuteri* as a probiotic strain to a subject, wherein *L. reuteri* is pre-conditioned, improved the muscle condition of the subject. The effect is enhanced by the presence of specific micronutrients in specific levels and by the presence of specific oils in the nutritional composition.

Accordingly, in a first aspect, the invention provides a nutritional composition comprising *L. reuteri* as a probiotic strain, wherein *L. reuteri* is pre-conditioned.

In a further aspect, the invention provides a nutritional composition comprising *L. reuteri* as a probiotic strain, wherein *L. reuteri* is pre-conditioned, for use in improving and/or increasing and/or maintaining and/or treating and/or stimulating and/or promoting at least one muscle condition in a subject.

In a further aspect, the invention provides the use of a nutritional composition comprising *L. reuteri* as a probiotic strain, wherein *L. reuteri* is pre-conditioned, for improving and/or increasing and/or maintaining and/or treating and/or stimulating and/or promoting at least one muscle condition in a subject.

In a further aspect, the invention provides a method of improving and/or increasing and/or maintaining and/or treating and/or stimulating and/or promoting at least one muscle condition in a subject, comprising a step of administrating a nutritional composition comprising *L. reuteri* as a probiotic strain, wherein *L. reuteri* is pre-conditioned to the subject.

In a further aspect, the invention provides a method or a process of using a nutritional composition comprising *L. reuteri* as a probiotic strain, wherein *L. reuteri* is pre-conditioned, for improving and/or increasing and/or maintaining and/or treating and/or stimulating and/or promoting at least one muscle condition in a subject, comprising a step of administrating the nutritional composition to the subject.

In a further aspect, the invention provides the use of a composition comprising *L. reuteri* as a probiotic strain, wherein *L. reuteri* is pre-conditioned, for the manufacture of a medicament or of a nutritional composition for improving and/or increasing and/or maintaining and/or treating and/or stimulating and/or promoting at least one muscle condition in a subject.

In some embodiments, *L. reuteri* is pre-conditioned with galacto-oligosaccharide (GOS), also called GOS pre-conditioned *L. reuteri.*

In some embodiments, the pre-conditioned *L. reuteri* is obtained or obtainable by a process comprising the following steps:
a) cultivating a probiotic *L. reuteri* strain in the presence of galacto-oligosaccharide (GOS) in a growth medium, thereby pre-conditioning the probiotic *L. reuteri* strain;
b) harvesting the pre-conditioned *L. reuteri* from the growth medium.

In some embodiments, the pre-conditioned *L. reuteri* is obtained or obtainable by a process comprising the following further step:
c) adding (fresh) GOS to the pre-conditioned probiotic *L. reuteri* strain obtained from step b).

The addition of (fresh) GOS as a prebiotic into the already pre-conditioned probiotic is increasing/boosting the synbiotic effect obtained by the pre-conditioning.

In some embodiments, the nutritional composition is further comprising at least one vitamin.

In some embodiments, the at least one vitamin is selected form the list consisting of: vitamin A, vitamin B1, vitamin B2, vitamin B3, vitamin B6, vitamin B12, vitamin C, vitamin D, vitamin E, vitamin K, vitamin B7, vitamin B5, vitamin B9, and any combinations thereof.

In some embodiments, the at least one vitamin is a combination comprising or consisting of high amounts of vitamin B3, vitamin C, vitamin D, vitamin E, vitamin K, vitamin B7, vitamin B5, vitamin B9, wherein the high amount are defined as being at least 30% more than in the draft of the Codex Standard For Follow-Up Formula For Older Infants And Product For Young Children CXS 156-1987 aged 6-36 months, report dated March 2023, appendix 2 from page 30, which is publicly available.

In some embodiments, the at least one vitamin is present in the following amount:
vitamin A, in an amount ranging of from 1000 IU / 100 g to 3000 IU / 100 g, preferably of from 1500 IU / 100 g to 2600 IU / 100 g, more preferably of from 2000 IU / 100 g to 2500 IU / 100 g, wherein one IU for vitamin A is the biological equivalent 0.3 microgram of retinol or of beta-carotene; and/or
vitamin B1, in an amount ranging of from 0.1 mg / 100 g to 1 mg / 100 g, preferably of from 0.3 mg / 100 g to 1 mg / 100 g, more preferably of from 0.5 mg / 100 g to 0.9 mg / 100 g; and/or
vitamin B2, in an amount ranging of from 1 mg / 100 g to 3 mg / 100 g, preferably of from 1 mg / 100 g to 2 mg / 100 g, more preferably of from 1.1 mg / 100 g to 1.6 mg / 100 g; and/or vitamin B3, in an amount ranging of from 1.5 mg / 100 g to 12 mg / 100 g, preferably of from 3 mg / 100 g to 10 mg / 100 g, more preferably of from 4 mg / 100 g to 8 mg / 100 g; and/or
vitamin B6, in an amount ranging of from 0 mg / 100 g to 1.3 mg / 100 g, preferably of from 0.2 mg / 100 g to 1 mg / 100 g, more preferably of from 0.3 mg / 100 g to 0.7 mg / 100 g; and/or
vitamin B12, in an amount ranging of from 0 microgram / 100 g to 3 microgram / 100 g, preferably of from 1 microgram / 100 g to 2.5 microgram / 100 g, more preferably of from 1 microgram / 100 g to 2 microgram / 100 g; and/or
vitamin C, in an amount ranging of from 80 mg / 100 g to 400 mg / 100 g, preferably of from 85 mg / 100 g to 300 mg / 100 g, more preferably of from 100 mg / 100 g to 200 mg / 100 g; and/or
vitamin D, in an amount ranging of from 130 IU / 100 g to 900 IU / 100 g, preferably of from 200 IU / 100 g to 700 IU / 100 g, more preferably of from 350 IU / 100 g to 500 IU / 100 g, wherein one IU for vitamin D is the biological equivalent of 0.025 microgram of cholecalciferol or ergocalciferol; and/or
vitamin E, in an amount ranging of from 11 IU / 100 g to 30 IU / 100 g, preferably of from 12 IU / 100 g to 25 IU / 100 g, more preferably of from 12 IU / 100 g to 20 IU / 100 g, wherein one IU for vitamin E is the biological equivalent of 0.67 mg of d-alpha-tocopherol, or of 0.9 mg of dl-alpha-tocopherol; and/or
vitamin K, in an amount ranging of from 23 microgram / 100 g to 100 microgram / 100 g, preferably of from 25 microgram / 100 g to 80 microgram / 100 g, more preferably of from 25 microgram / 100 g to 70 microgram / 100 g; and/or
vitamin B7, in an amount ranging of from 9 microgram / 100 g to 100 microgram / 100 g, preferably of from 12 microgram / 100 g to 60 microgram 100 g, more preferably of from 15 microgram / 100 g to 30 microgram / 100 g; and/or
B5, in an amount ranging of from 2.1 mg / 100 g to 10 mg / 100 g, preferably of from 2.5 mg / 100 g to 8 mg / 100 g, more preferably of from 3 mg / 100 g to 6 mg / 100 g; and/or
vitamin B9, in an amount ranging of from 51 microgram / 100 g to 350 microgram / 100 g, preferably of from 70 microgram / 100 g to 300 microgram / 100 g, more preferably of from 90 microgram / 100 g to 200 microgram / 100 g.

In some embodiments, the at least one vitamin is present in the following amount:
vitamin A, in an amount ranging of from 200 IU / 100 kcal to 1000 IU / 100 kcal, preferably of from 300 IU / 100 kcal to 800 IU / 100 kcal, more preferably of from 300 IU / 100 kcal to 600 IU / 100 kcal, wherein one IU for vitamin A is the biological equivalent 0.3 microgram of retinol or of beta-carotene; and/or
vitamin B1, in an amount ranging of from 0.02 mg / 100 kcal to 0.5 mg / 100 kcal, preferably of from 0.05 mg / 100 kcal to 0.4 mg / 100 kcal, more preferably of from 0.05 mg / 100 kcal to 0.2 mg / 100 kcal; and/or
vitamin B2, in an amount ranging of from 0.02 mg / 100 kcal to 0.6 mg / 100 kcal, preferably of from 0.05 mg / 100 kcal to 0.5 mg / 100 kcal, more preferably of from 0.08 mg / 100 kcal to 0.5 mg / 100 kcal; and/or
vitamin B3, in an amount ranging of from 0.3 mg / 100 kcal to 3 mg / 100 kcal, preferably of from 0.6 mg / 100 kcal to 2.5 mg / 100 kcal, more preferably of from 0.8 mg / 100 kcal to 2 mg / 100 kcal; and/or
vitamin B6, in an amount ranging of from 0 mg / 100 kcal to 0.3 mg / 100 kcal, preferably of from 0.05 mg / 100 kcal to 0.25 mg / 100 kcal, more preferably of from 0.05 mg / 100 kcal to 0.2 mg / 100 kcal; and/or
vitamin B12, in an amount ranging of from 0 microgram / 100 kcal to 0.65 microgram / 100 kcal, preferably of from 0.05 microgram / 100 kcal to 0.6 microgram / 100 kcal, more preferably of from 0.05 microgram / 100 kcal to 0.5 microgram / 100 kcal; and/or
vitamin C, in an amount ranging of from 17 mg / 100 kcal to 100 mg / 100 kcal, preferably of from 20 mg / 100 kcal to 80 mg / 100 kcal, more preferably of from 20 mg / 100 kcal to 50 mg / 100 kcal; and/or
vitamin D, in an amount ranging of from 26 IU / 100 kcal to 150 IU / 100 kcal, preferably of from 40 IU / 100 kcal to 120 IU / 100 kcal, more preferably of from 50 IU / 100 kcal to 130 IU / 100 kcal, wherein one IU for vitamin D is the biological equivalent of 0.025 microgram of cholecalciferol or ergocalciferol; and/or
vitamin E, in an amount ranging of from 2.0 IU / 100 kcal to 12 IU / 100 kcal, preferably of from 2.5 IU / 100 kcal to 10 IU / 100 kcal, more preferably of from 2.6 IU / 100 kcal to 5 IU / 100 kcal, wherein one IU for vitamin E is the biological equivalent of 0.67 mg of d-alpha-tocopherol, or of 0.9 mg of dl-alpha-tocopherol; and/or
vitamin K, in an amount ranging of from 4.6 microgram / 100 kcal to 25 microgram / 100 kcal, preferably of from 5 microgram / 100 kcal to 20 microgram / 100 kcal, more preferably of from 5 microgram / 100 kcal to 15 microgram / 100 kcal; and/or
vitamin B7, in an amount ranging of from 2 microgram / 100 kcal to 15 microgram / 100 kcal, preferably of from 2.5 microgram / 100 kcal to 12 microgram / 100 kcal, more preferably of from 3 microgram / 100 kcal to 10 microgram / 100 kcal; and/or
B5, in an amount ranging of from 0.43 mg / 100 kcal to 2 mg / 100 kcal, preferably of from 0.5 mg / 100 kcal to 1.6 mg / 100 kcal, more preferably of from 0.55 mg / 100 kcal to 1.2 mg / 100 kcal; and/or
vitamin B9, in an amount ranging of from 13 microgram / 100 kcal to 80 microgram / 100 kcal, preferably of from 15 microgram / 100 kcal to 60 microgram / 100 kcal, more preferably of from 20 microgram / 100 kcal to 45 microgram / 100 kcal.

In some embodiments, the nutritional composition is comprising high amounts of the following at least one vitamin, wherein the high amounts are preferably defined as above:
a) vitamin B3;
b) vitamin D;
c) vitamin B9 ;
d) vitamin B3, vitamin D and vitamin B9;
e) vitamin B3 and vitamin D;
f) vitamin B3 and vitamin B9;
g) vitamin D and vitamin B9;
h) vitamin B3 and vitamin B7;
i) vitamin D and vitamin B7;
j) vitamin B9 and vitamin B7;
k) vitamin B3, vitamin D and vitamin B7;
l) vitamin B3, vitamin D, vitamin B9 and vitamin B7;
m) vitamin B3, vitamin D and vitamin K;
n) vitamin B3, vitamin D, vitamin B7 and vitamin K;
o) vitamin B3, vitamin D, vitamin B7, vitamin B9 and vitamin K;
p) vitamin B3, vitamin D, vitamin B7, vitamin B9, vitamin C and vitamin K;
q) vitamin B3, vitamin D, vitamin B7, vitamin B9, vitamin C, vitamin E and vitamin K;
r) vitamin D and vitamin B5;
s) vitamin B9 and vitamin B5;
t) vitamin B3, vitamin D, vitamin B5 and vitamin B9;
u) vitamin B3, vitamin D and vitamin B5;
v) vitamin B3, vitamin B9 and vitamin B5;
w) vitamin D, vitamin B9 and vitamin B5;
x) vitamin B3, vitamin D, vitamin B7, vitamin B9, vitamin C, vitamin E, vitamin K and vitamin B5.

In some preferred embodiments, the nutritional composition is comprising high amounts of the following at least one vitamin, wherein the high amounts are preferably defined as above:
- vitamin B3;
- vitamin D;
- vitamin B9;
- vitamin B3, vitamin D and vitamin B9;
- vitamin B3 and vitamin D;
- vitamin B3 and vitamin B9;
- vitamin D and vitamin B9;
- vitamin B3 and vitamin B7;
- vitamin D and vitamin B7;
- vitamin B9 and vitamin B7;
- vitamin B3, vitamin D and vitamin B7;
- vitamin B3, vitamin D, vitamin B9 and vitamin B7;
- vitamin B3, vitamin D and vitamin K;
- vitamin B3, vitamin D, vitamin B7 and vitamin K.

In some preferred embodiments, the nutritional composition is comprising high amounts of the following at least one vitamin, wherein the high amounts are preferably defined as above:
- vitamin D;
- vitamin B3, vitamin D and vitamin B9;
- vitamin B3 and vitamin D;
- vitamin D and vitamin B9;
- vitamin D and vitamin B7;
- vitamin B3, vitamin D and vitamin B7;
- vitamin B3, vitamin D, vitamin B9 and vitamin B7;
- vitamin B3, vitamin D and vitamin K;
- vitamin B3, vitamin D, vitamin B7 and vitamin K.

In some embodiments, the nutritional composition is further comprising lipids comprising fatty acid (FA) triacylglycerols (TAG).

In some embodiments, in said lipids comprising fatty acid (FA) triacylglycerols (TAG), said fatty acid TAG comprise stearic, palmitic and myristic esters, wherein the sum of the amount of stearic, palmitic and myristic esters at the sn-1(3) position of the TAG is less than 13.0 wt-% of TAG and the myristic acid represents up to 3.7 wt-% of the total FA, the palmitic acid represents up to 11.1 wt-% of the total FA and palmitic acid represents up to 10.0 wt-% of the total FA, wherein the SCFA (short chain fatty acids) being are present in preferably less than 40 wt-%, wherein the MUFA (mono-unsaturated fatty acids) are present preferably more than 35.1 %.

In some embodiments, said lipids comprise a mixture of oils selected from:
16.9 wt-% to 22.0 wt-% of rapeseed oil, 11.5 to 14.5 wt-% of sunflower oil, 40.0 to 58 wt-% of high oleic sunflower oil, and 15.0 wt-% to 20.5 wt-% of coconut oil.

In some embodiments, the nutritional is further comprising at least one mineral.

In some embodiments, the at least one mineral is selected form the list consisting of: calcium, phosphorous, magnesium, iron, zinc, potassium, copper, manganese, selenium, sodium and any combinations thereof.

In some embodiments, the at least one mineral is selenium, manganese, a combination comprising or consisting of selenium, manganese, or a combination comprising or consisting of magnesium, manganese, selenium.

In some embodiments, the at least one vitamin is a combination comprising or consisting of high amounts of manganese and selenium, wherein the high amount are defined as being at least 30% more than in the draft of the Codex Standard For Follow-Up Formula For Older Infants And Product For Young Children CXS 156-1987 aged 6-36 months, report dated March 2023, appendix 2 from page 30, which is publicly available.

In some embodiments, the at least one mineral is present in the following amount: magnesium, in an amount ranging of from 30 mg / 100 g to 200 mg / 100 g, preferably of from 40 mg / 100 g to 150 mg / 100 g, more preferably of from 15 mg / 100 g to 150 mg / 100 g; and/or
manganese, in an amount ranging of from 70 microgram / 100 g to 800 microgram / 100 g, preferably of from 100 microgram / 100 g to 450 microgram / 100 g, more preferably of from 100 microgram / 100 g to 250 microgram / 100 g; and/or
selenium, in an amount ranging of from 7 microgram / 100 g to 120 microgram / 100 g, preferably of from 10 microgram / 100 g to 50 microgram / 100 g, more preferably of from 10 microgram / 100 g to 25 microgram / 100 g;
   and/or
magnesium in an amount ranging of from 6 mg / 100 kcal to 30 mg / 100 kcal, preferably of from 8 mg / 100 kcal to 25 mg / 100 kcal, more preferably of from 10 mg / 100 kcal to 15 mg / 100 kcal; and/or
manganese in an amount ranging of from 15 microgram / 100 kcal to 250 microgram / 100 kcal, preferably of from 20 microgram / 100 kcal to 200 microgram / 100 kcal, more preferably of from 20 microgram / 100 kcal to 70 microgram / 100 kcal; and/or
selenium in an amount ranging of from 2.5 microgram / 100 kcal to 12 microgram / 100 kcal, preferably of from 2.8 microgram / 100 kcal to 9 microgram / 100 kcal, more preferably of from 3.0 microgram / 100 kcal to 9 microgram / 100 kcal.

In some embodiments, the nutritional composition is further comprising at least one further probiotic.

In some embodiments, the at least one further probiotic is a *Bifidobacterium.*

In some embodiments, the at least one further probiotic is *Bifidobacterium* selected form the list consisting of: *Bifidobacterium dentium, Bifidobacterium infantis,* and any combinations thereof.

In some embodiments, the at least one further probiotic is a *Limosilactobacillus.*

In some embodiments, the at least one further probiotic is *Dorea phocaeensis.*

In some embodiments, the nutritional composition is for use in increasing the abundance of at least one probiotic in the gut of a subject. In some preferred embodiments, the at least one probiotic is selected form the list consisting of a *Dorea, a Bifidobacterium,* and any combination thereof. In some preferred embodiments, the at least one probiotic is a *Bifidobacterium* selected form the list consisting of: *Bifidobacterium dentium, Bifidobacterium infantis,* and combination thereof. In some preferred embodiments, the at least one probiotic is a *Limosilactobacillus.* In some preferred embodiments, the at least one probiotic is *Dorea phocaeensis.* In some embodiments, the nutritional composition in for use in increasing the abundance of at least one probiotic in the gut of a subject compared to a subject not having consumed the nutritional composition according to the invention.

In some embodiments, the nutritional composition is used in a method of increasing the abundance of at least one probiotic in the gut of a subject, the method comprising a step of administering the nutritional composition to the subject. In some preferred embodiments, the at least one probiotic is selected form the list consisting of a *Dorea, a Bifidobacterium,* and any combination thereof. In some preferred embodiments, the at least one probiotic is a *Bifidobacterium* selected form the list consisting of: *Bifidobacterium dentium, Bifidobacterium infantis,* and combination thereof. In some preferred embodiments, the at least one probiotic is a *Limosilactobacillus.* In some preferred embodiments, the at least one probiotic is *Dorea phocaeensis.* In some embodiments, the nutritional composition in for use in increasing the abundance of at least one probiotic in the gut of a subject compared to a subject not having consumed the nutritional composition according to the invention.

In one embodiment, the method of increasing the abundance of at least one probiotic in the gut of a subject is non-therapeutic.

In some embodiments, the nutritional composition is an infant formula, a starter infant formula, a follow-on or follow-up infant formula, a baby food, an infant cereal composition, a growing-up milk, a fortifier or a supplement.

In some embodiments, the subject is an infant.

In some embodiments, the subject is a young child.

In some embodiments, the subject is a child.

In some embodiments, the subject was born by C- section and/or preterm, and/or small for gestational age (SGA) and/or with a low, very low, or extremely low weight at birth (LWB, VLBW, or ELBW) and/or experienced intra-uterine growth retardation (IUGR). Those subjects usually have a low muscle mass.

In some embodiments, the nutritional composition is used, for use or used in a method in or for improving and/or increasing and/or maintaining and/or promoting and/or treating and/or preventing at least one muscle condition.

In some embodiments, the nutritional composition is used, for use or used in a method in or for improving and/or increasing and/or maintaining at least one muscle condition.

In some embodiments, the nutritional composition is used, for use or used in a method in or for improving and/or increasing at least one muscle condition.

In some embodiments, the nutritional composition is used, for use or used in a method in or for improving at least one muscle condition.

In some embodiments, the nutritional composition is for use in treating a muscle disorder in a subject.

In some embodiments, the nutritional composition is for use in preventing a muscle disorder in a subject.

In some embodiments, the nutritional composition is for use in treating a muscle disorder in a subject, wherein the subject is infant, a young child or a child.

In some embodiments, the nutritional composition is for use in preventing a muscle disorder in a subject infant, a young child or a child.

In some embodiments, the nutritional composition is used, for use or used in a method in or for improving and/or increasing and/or maintaining and/or treating and/or stimulating and/or promoting and/or preventing at least one muscle condition in a subject, such as infant, a young child or a child, selected form the list consisting of:
increasing muscle strength;
increasing muscle force;
increasing grip strength;
improving endurance capacity;
increasing muscle quality;
increasing or maintaining muscle mass;
increasing muscle specific force;
increasing exercise capacity;
modulating muscle health;
increasing muscle force generation;
treating a muscle disorder;
preventing a muscle disorder;
promoting muscle health;
promoting normal muscle function;
modulating muscle stem cell function;
promoting muscle stem cell function;
increasing muscle stem cell function;
and any combinations thereof;

In some embodiments, the nutritional composition is used, for use or used in a method in or for increasing or maintaining muscle strength and/or muscle mass.

In some embodiments, the nutritional composition is used, for use or used in a method in or for increasing muscle strength.

In some embodiments, the nutritional composition is used, for use or used in a method in or for increasing or maintaining muscle mass.

In some embodiments, the nutritional composition is used, for use or used in a method in or for increasing muscle force and/or increasing grip strength.

In some embodiments, the nutritional composition is used, for use or used in a method in or for increasing grip strength.

In some embodiments, the nutritional composition is used, for use or used in a method in or for increasing muscle force.

In some embodiments, the nutritional composition is used, for use or used in a method in or for the maintenance of or increase in muscle function such as normal muscle function and/or muscle mass.

### Brief description of the Figures

**Figure 1****:** Handgrip of subjects treated with EXPL of the invention and CTRL formulae.
**Figure 2****:** Gut microbiota analysis for microbiome composition and diversity for REF, CTRL and REF fed groups.
**Figure 3****:** Relative abundance of L reuteri in the feces of subjects in EXPL (YCF) compared to CTRL groups (Control), abundance of *L. reuteri.*
**Figure 4****:** heatmap summarizing correlations between clinical variables and microbiome taxa at species level in all the subjects from all the groups together.
**Figure 5****:** Serum Vitamin D distribution at Visit 1 (Baseline) and Visit 3 (6 months of intervention) in the ITT for EXPL (YCF), CTRL and REF groups.

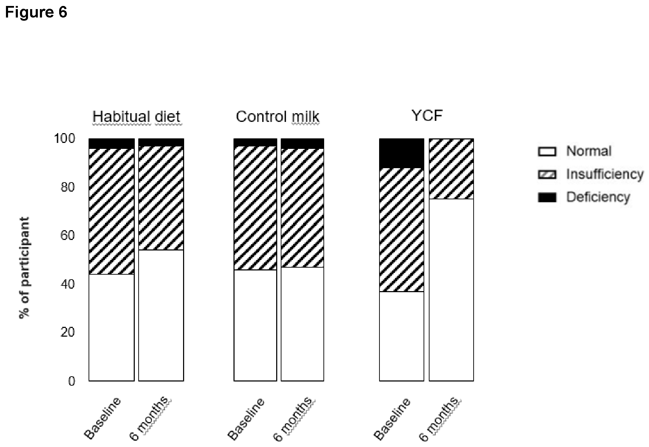

**Figure 6****.** Prevalence of Vitamin D insufficiency and deficiency at Visit 1 (Baseline) and Visit 3 (6 months of intervention) in the FAS for REF (Habitual diet), CTRL (Control milk) and EXPL (YCF) groups.
**Figure 7****.** Level of Vitamin B9 (5-methyl tetrahydrofolate) in the plasma at Visit 1 (Baseline) and Visit 3 (6 months of intervention) for REF (Habitual diet), CTRL (Control milk) and EXPL (YCF) groups.

### Detailed description of the invention

### Definitions

All percentages are by weight unless otherwise stated.

All ingredient units (g, mg, microgram, IU...) /100g are /100g of the nutritional composition in a solid (e.g. powdered) form, unless otherwise stated.

The terms "about" or "approximatively" as used herein when referring to a measurable value such as a parameter, an amount, a temporal duration, and the like, are meant to encompass variations of and from the specific value, such as the variation of 1/-10% or less, 1/-5% or less, 1/-1% or less, and +/0.1% or less of and from the specific value, insofar such variations are appropriate to perform in the disclosed invention. It is to be understood that the value to which the modifier "about" or "approximately" refers is itself also specifically, and preferably, disclosed.

The term "subject" refers to a vertebrate, preferably a mammal, more preferably a human, more particularly an infant, a young child or a child. Mammals include but are not limited to murines, simians, humans, farm animals, sport animals and pets.

The term "infant" means a child under the age of 12 months.

The terms "young child" or "toddler" as used herein may mean a human subject aged between 12 months and 5 years of age.

The term "child" means a child aged between three and twelve years. Preferably, the term "child" means a child aged between three and six years.

A "preterm" or "premature" subject means an infant or young child who was not born at term. Generally it refers to an infant or young child born prior 36 weeks of gestation.

By the expression "small for gestational age" or "SGA" it is referred to an infant or young child who is smaller in size than normal for their gestational age at birth, most commonly defined as a weight below the 10th percentile for the gestational age. In some embodiments, SGA may be associated with intrauterine growth restriction (IUGR), which refers to a condition in which a foetus is unable to achieve its potential size.

A "preterm" or "premature" means an infant or young child who was not born at term. Generally, it refers to an infant or young child born prior 37 weeks of gestation.

An "infant having a low birth weight" means a preterm having a body weight below 2500g (5.5 pounds) either because of preterm birth or restricted fetal growth. It therefore encompasses:
- infant or young child who has/had a body weight from 1500 to 2500 g at birth (usually called "low birth weight" or LBW)
- infant or young child who has/had a body weight from 1000 to 1500 g at birth (called "very low birth weight" or VLBW)
- infant or young child who has/had a body weight under 1000 g at birth (called "extremely low birth weight" or ELBW).

The expressions "composition" or "nutritional composition" refer to any kind of composition or formulation that provides a nutritional benefit to an individual and that may be safely consumed by a human or an animal. A nutritional composition may be in solid (e.g. powder), semi-solid or liquid form and may comprise one or more macronutrients, micronutrients, food additives, water, etc. For instance, the nutritional composition may comprise the following macronutrients: a source of proteins, a source of lipids, a source of carbohydrates and any combination thereof. Furthermore, the nutritional composition may comprise the following micronutrients: vitamins, minerals, fiber, phytochemicals, antioxidants, prebiotics, probiotics, bioactives, metabolites (e.g. butyrate, Docosahexaenoic acid (DHA), Eicosapentaenoic acid (EPA), Gamma-Linolenic acid (GLA)) and any combination thereof. The composition may also contain food additives such as stabilizers (when provided in liquid or solid form) or emulsifiers (when provided in liquid form). The amount of the various ingredients (e.g. the prebiotics, vitamins, minerals, proteins, carbohydrates, fat/lipid etc.) can be expressed in g/100 g or IU/100g of composition on a dry weight basis when it is in a solid form, e.g. a powder, or as a concentration in g/L of the composition when it refers to a liquid form (this latter also encompasses liquid composition that may be obtained from a powder after reconstitution in a liquid such as milk, water, e.g. a reconstituted infant formula or follow-on/follow-up formula or infant cereal product or any other formulation designed for infant or young child nutrition). Generally, a nutritional composition can be formulated to be taken enterally, orally, parenterally, or intravenously, and it usually includes one of more nutrients selected from: a lipid or fat source, a protein source, and a carbohydrate source. Preferably, a nutritional composition is for oral use. In a particular embodiment the nutritional composition is a ready-to-drink composition such as a ready-to-drink formula.

In a particular embodiment, the nutritional composition of the present invention is a "synthetic nutritional composition". The expression "synthetic nutritional composition" means a mixture obtained by chemical and/or biological means, which can be chemically identical to the mixture naturally occurring in mammalian milks (i.e. the synthetic nutritional composition is not breast milk).

The expression "infant formula" as used herein refers to a foodstuff intended for particular nutritional use by infants during the first months of life and satisfying by itself the nutritional requirements of this category of person (Article 2(c) of the European Commission Directive 91/321/EEC 2006/141/EC of 22 December 2006 on infant formulae and follow-on formulae). It also refers to a nutritional composition intended for infants and as defined in Codex Alimentarius (Codex STAN 72-1981) and Infant Specialities (incl. Food for Special Medical Purpose).

The expression "infant formula" encompasses both "starter infant formula" and "follow-up formula" or "follow-on formula".

A "follow-up formula" or "follow-on formula" is given from the 6th month onwards and includes "growing-up milk". It constitutes the principal liquid element in the progressively diversified diet of this category of person.

The expression "growing-up milk" (or "GUM") refers to a milk-based drink generally with added vitamins and minerals, that is intended for young children or children.

The expression "baby food" means a foodstuff intended for particular nutritional use by infants or young children during the first years of life.

The expression "infant cereal composition" means a foodstuff intended for particular nutritional use by infants or young children during the first years of life.

The terms "fortifier" refers to liquid or solid nutritional compositions suitable for fortifying or mixing with human milk, infant formula, growing-up milk or human breast milk fortified with other nutrients. Accordingly, the fortifier can be administered after dissolution in human breast milk, in infant formula, in growing-up milk or in human breast milk fortified with other nutrients or otherwise it can be administered as a stand-alone composition. When administered as a stand-alone composition, the milk fortifier can be also identified as being a "supplement".

The term "metabolize" is used herein to mean that a substrate can by broken down, adsorbed and/or utilized by a microorganism. For example, the substrate may promote and/or contribute to the growth and/or survival of the microorganism.

The expression "weaning period" means the period during which the mother's milk is substituted by other food in the diet of an infant or young child.

The "mother's milk" should be understood as the breast milk including colostrum (first milk), transitional or mature milk of the mother.

The "oligosaccharide" may refer to a carbohydrate that has greater than 2 but relatively few monosaccharide units (typically 3, 4, 5, 6, and up to 10). Exemplary oligosaccharides include, but are not limited to, fructo-oligosaccharides, galacto-oligosaccharides (raffinose, stachyose, verbascose), maltooligosaccharides, gentio-oligosaccharides, cellooligosaccharides, milk oligosaccharides (e.g., those present in secretions from mammary glands), isomalto-oligosaccharides, lactosucrose, mannooligosaccharides, melibiose-derived oligosaccharides, pectic oligosaccharides, xylo-oligosaccharides.

The term "polysaccharide" may refer to a carbohydrate that has more than ten monosaccharide units. Exemplary polysaccharides include, but are not limited to, starch, arabinogalactan, laminarin, chrysolaminarin, xylan, arabinoxylan, mannan, fucoidan and galactomannan. It is to be understood that there is not a precise cut-off or distinction between the terms oligosaccharide and polysaccharide, nor is such a distinction necessary to practice the invention.

The term "GOS" as used herein means "galacto-oligosaccharide". Galacto-oligosaccharides (GOS) as used herein typically consist of β-linked galactose moieties with galactose or glucose at the reducing end. Such GOS contains β-(1→2), β-(1→3), β-(1→4), or β-(1→6) linked galactose moieties and may have a degree of polymerization (DP) of 3-8 galactose units. The term GOS is therefore preferably referred to as oligosaccharide(s) comprising at least three galactose units, more preferably as oligosaccharide(s) comprising at least four galactose units, preferably having a degree of polymerization (DP) of 3-8 galactose units.

The term "HMO" or "HMOs" refers to human milk oligosaccharide(s). These carbohydrates are highly resistant to enzymatic hydrolysis, indicating they may display essential functions not directly related to their caloric value. It has been especially illustrated they play a vital role in the early development of infants and young children, such as the maturation of the immune system. Many different kinds of HMOs are found in the human milk. Each individual oligosaccharide is based on a combination of glucose, galactose, sialic acid (N-acetylneuraminic acid), fucose and/or N-acetylglucosamine with many and varied linkages between them, thus accounting for the enormous number of different oligosaccharides in human milk - over 130 such structures have been identified so far. Almost all of them have a lactose moiety at their reducing end while sialic acid and/or fucose (when present) occupy the terminal position at the non-reducing ends. Depending on the presence of fucose and sialic acid in the oligosaccharide structure, the HMOs can be divided as non-fucosylated (neutral) or fucosylated (neutral) and sialylated (acidic) and non-sialylated molecules, respectively.

The expression "fucosylated oligosaccharide" refers to an oligosaccharide having a fucose residue. It has a neutral nature. Some examples are 2'-fucosyllactose (2'-FL), 3-fucosyllactose (3-FL), difucosyllactose (DiFL), lacto-N-fucopentaose (e.g. lacto-N-fucopentaose I, lacto-N-fucopentaose II, lacto-N-fucopentaose III, lacto-N-fucopentaose V), lacto-N-fucohexaose, lacto-N-difucohexaose I, fucosyllacto-N-hexaose, fucosyllacto-N-neohexaose, difucosyllacto-N-hexaose I, difucosyllacto-N-neohexaose II and any combination thereof. Fucosylated oligosaccharides represents the largest fraction of human milk with 2'-FL constituting up to 30% of the total HMOs. Fucosylated oligosaccharides are thought to reduce the risk of infections and inflammations and to boost growth and metabolic activity of specific commensal microbes reducing inflammatory response.

The expression "N-acetylated oligosaccharide(s)" encompasses both "N-acetyl-lactosamine" and "oligosaccharide(s) containing N-acetyl-lactosamine". They are neutral oligosaccharides having an N-acetyl-lactosamine residue. Suitable examples are LNT (lacto-N-tetraose), para-lacto-N-neohexaose (para-LNnH), LNnT (lacto-N-neotetraose), DSLNT (disialyllacto-N-tetraose), and any combinations thereof. Other examples are lacto-N-hexaose, lacto-N-neohexaose, para- lacto-N-hexaose, para-lacto-N-neohexaose, lacto-N-octaose, lacto-N-neooctaose, iso- lacto-N-octaose, para- lacto-N-octaose and lacto-N-decaose.

The expressions "at least one fucosylated oligosaccharide" and "at least one N-acetylated oligosaccharide" should be understood as "at least one type of fucosylated oligosaccharide" and "at least one type of N-acetylated oligosaccharide".

The term "sialylated oligosaccharide" refers to an oligosaccharide having a charged sialic acid residue. It has an acidic nature. Some examples are 3'-sialyllactose (3-SL), 6'-sialyllactose (6-SL), sialyllacto-N-tetraose (Lst - e.g. Lst-a, Lst-b or Lst-c).

Suitably, the term "capable of metabolizing the HMO" may mean that the *B. longum* transitional strain encodes at least one CAZyme which is capable of utilizing the HMO. For example, the CAZyme may be capable of catalyzing the hydrolysis of a glycosidic bond within the HMO. Suitably, the *B. longum* transitional strain may encode at least one, at least two, at least three, at least four or at least five CAZymes that are capable of utilizing the HMO. Suitably, the term "capable of metabolizing the HMO" may mean that the HMO is capable of promoting growth and/or survival of the *B. longum* transitional strain (e.g. when added to an anaerobic culture of the *B. longum* transitional strain). Growth and/or survival of the *B. longum* transitional strain may be determined by measuring the abundance of 16S rDNA - for example using PCR methods.

The term fibers is used herein to refer to carbohydrates that are indigestible by a human or animal. Such fibers are also discussed in relation to carbohydrates herein. Suitably, the fiber can be fermented by one or more *B. longum* transitional microorganisms provided in the present use or composition and/or within one or more regions in the gastrointestinal tract within an organism, such as a human or non-human animal. As used herein, the expressions "fiber" or "fibers" or "dietary fiber" or "dietary fibers" within the context of the present invention indicate the indigestible portion, in small intestine, of food derived from plants which comprises two main components: soluble fiber, which dissolves in water and insoluble fiber. Mixtures of fibers are comprised within the scope of the terms above mentioned. Soluble fiber is readily fermented in the colon into gases and physiologically active byproducts and can be prebiotic and viscous. Insoluble fiber does not dissolve in water, is metabolically inert and provides bulking, or it can be prebiotic and metabolically ferment in the large intestine. Chemically, dietary fiber consists of carbohydrate polymers with three or more monomeric units which are not hydrolyzed by endogenous enzymes in the small intestine such as arabinoxylans, cellulose, and many other plant components such as resistant starch, resistant dextrins, inulin, lignin, chitins, pectins, arabinans, arabinogalactans, galactans, xylans, beta-glucans, and oligosaccharides. Non-limiting examples of dietary fibers are: prebiotic fibers such as Fructo-oligosaccharides (FOS), inulin, galacto-oligosaccharides (GOS), fruit fiber, vegetable fiber, cereal fiber, resistant starch such as high amylose corn starch.

As used herein, "added fiber" or "added dietary fiber" indicates an ingredient mainly or totally constituted by fiber which is added to the complementary nutritional composition and whose content in fiber contributes to the total fiber content of the composition. The total fiber content of the complementary nutritional composition is provided by the sum of amount of fiber naturally present in ingredients used in the recipe (for example from whole grain cereal flour) plus amount of added fiber.

The term "prebiotic" means non-digestible carbohydrates that beneficially affect the host by selectively stimulating the growth and/or the activity of healthy bacteria such as bifidobacteria in the colon of humans (Gibson GR, Roberfroid MB. Dietary modulation of the human colonic microbiota: introducing the concept of prebiotics. J Nutr. 1995;125:1401-12).

The term "probiotic" means microbial cell preparation or components of microbial cells with a beneficial effect on the health or well-being of the host (Salminen S, Ouwehand A. Benno Y. et al. "Probiotics: how should they be defined" Trends Food Sci. Technol. 1999:10 107-10). The microbial cells according to the present invention are generally bacteria.

The term "cfu" should be understood as colony forming unit.

The term "pre-conditioned" in the context of the present invention also means "pre-cultivated", "pre-activated", "pre-fermenting" *L. reuteri* as a probiotic strain preferably means growing, such as cultivating or fermenting, the probiotic with a prebiotic such as GOS during the process of producing the probiotic. The probiotic which has been pre-conditioned has an advantage when administered with the prebiotic over the indigenous or other non-preconditioned probiotic, because it is better capable of accessing the prebiotic later.

The "gut microbiota" is the composition of microorganisms (including bacteria, archaea and fungi) that live in the digestive tract.

The term "gut microbiome" may encompass both the "gut microbiota" and their "theater of activity", which may include their structural elements (nucleic acid, proteins, lipids, polysaccharides), metabolites (signaling molecules, toxins, organic and inorganic molecules) and molecules produced by coexisting hosts and structured by the surrounding environmental conditions (Berg, G., et al., 2020. Microbiome, 8(1), pp.1-22).

The term "SCFA" means short chain fatty acid(s).

The term "MUFA" means monounsaturated fatty acid(s).

The term "muscle stem cell", as used herein, may refer to satellite cells, preferably satellite cells that are quiescent and are uncommitted, as well as their activated, proliferating and differentiating progenies.

Satellite cells are precursors to skeletal muscle cells. In adult muscle, satellite cells are generally quiescent, but can activate and undergo myogenesis in response to disease or mechanical strain such as injury or exercise. Satellite cells are also involved in the normal growth of muscle. Upon activation, satellite cells proliferate before undergoing myogenic differentiation to finally fuse with existing myofibers or to form new myofibers, depending on the magnitude of tissue trauma. In addition to generating differentiated myogenic progeny, at least some satellite cells can self-renew, thereby meeting the defining criteria of bona fide resident stem cells.

MyoD+ is a commitment marker that may be used to distinguish quiescent from committed satellite cells.

The term "muscle function" refers to the ability of a muscle to perform in a manner that does not negatively impact on the life of a subject, and encompasses parameters of muscle strength, muscle contraction, muscle endurance, muscle elasticity, ability of a muscle to resist muscle fatigue and/or physical activities of daily living such as walking upstairs, getting out of a chair and other activities of daily living.

Suitable tests for assessing muscle function include grip strength assessment using a dynamometer; one repeat maximum on leg press, chest press or leg extension; gait speed; 6 min walk test; time up and go; short physical performance battery; Fried frailty criteria; and stair climbing time assessments. Other suitable tests include muscle strength, endurance and time to fatigue.

Muscle mass (which may equate with muscle volume, muscle thickness or myofiber size) may be measured by dual-energy X-ray absorptiometry (DXA) or bioimpedance tests. Similarly, MRI may be used for assessing muscle volume and ultra-sound may be used for assessing muscle thickness and pennation angle.

Muscle wasting may be a reduction in muscle mass, for example to the stage where the muscle loss becomes debilitating. In one embodiment, the subject does not lose more than 10%, 5%, 4%, 3%, 2% or 1% of their muscle mass.

The term "maintains" refers to a particular parameter, such as muscle function and/or mass, remaining substantially unchanged over a period of time (e.g. 5, 10, 15, 20, 25, 30, 40, 50 or more years).

In one embodiment, muscle mass increases by at least 1%, 2%, 3%, 4%, 5%, 10%, 15% or 20%.

In another embodiment, muscle mass increases by 1-2.5%, 1-5%, 1-10% or 1-20%.

Preferably, the muscle is skeletal muscle.

In one embodiment of the present invention, as a result of maintenance of or increase in muscle function and/or mass, the compounds, compositions, uses and methods disclosed herein provide for promotion of muscle growth and/or prevention of sub-optimal muscle growth in a subject, such as an infant, young child or child.

Within the context of the present invention the term "muscle growth" encompasses an increase of muscle mass, muscle volume and/or-muscle function, through time. Muscle growth may be evaluated by measuring muscle mass and/or function as above described.

Muscle growth can also be determined by an increase in muscle fiber number (hyperplasia) as well as an increase in muscle fiber size (hypertrophy).

Within the context of the present invention, the term "promoting" indicates a factor or a number of factors causing a certain process to occur.

All combinations of all ranges given can be combined according to the invention. That means that any broadest range can be combined with any preferred range, any broadest range can be combined with any more/most preferred range, any preferred range can be combined with any more/most preferred range, any alternative range can be combined with any broadest, preferred or more/most preferred range, etc.. In more preferred embodiments, the broadest ranges are combined with each other. In more preferred embodiments, the preferred ranges are combined with each other. In more preferred embodiments, the more/most preferred ranges are combined with each other.

In some embodiments the methods and uses in improving and/or increasing and/or maintaining and/or stimulating and/or promoting at least one muscle condition in a subject according to the present invention are therapeutic or non-therapeutic.

### Muscle condition

As exemplified above, handgrip is associated with several muscle conditions, of which, but not limited to
increasing muscle strength;
increasing muscle force;
increasing grip strength;
improving endurance capacity;
increasing muscle quality;
increasing or maintaining muscle mass;
increasing muscle specific force;
increasing exercise capacity;
modulating muscle health;
increasing muscle force generation;
treating a muscle disorder;
preventing a muscle disorder;
promoting muscle health;
promoting normal muscle function;
modulating muscle stem cell function;
promoting muscle stem cell function;
increasing muscle stem cell function.

### Muscle disorder

A muscle disorder is a condition that affects the structure and/or function of skeletal muscles, leading to symptoms such as muscle weakness, atrophy, pain, or abnormal muscle tone. These disorders can be genetic, autoimmune, inflammatory, or acquired through injury, infection or traumatic event (REF 1-5). Non-limiting examples of muscle disorder include muscle injury, nerve injury, muscular dystrophies (such as Duchenne muscular dystrophy, Becker muscular dystrophy, limb girdle muscular dystrophy, distal muscular dystrophy, myotonic muscular dystrophy), myopathies (such as Congenital Myopathies, Mitochondrial Myopathies, Inflammatory Myopathies, and Toxic Myopathies), cerebral palsy, cachexia, sarcopenia, frailty, muscle fatigue or muscle weakness, or impairment in one or more of muscle functionality, muscle performance, or muscle strength, neurodegenerative disorder that cause muscle weakness or atrophy (Dowling JJ, Weihl CC, Spencer MJ. Molecular and cellular basis of genetically inherited skeletal muscle disorders. Nat Rev Mol Cell Biol. 2021 Nov;22(11):713-732. doi: 10.1038/s41580-021-00389-z. Epub 2021 Jul 13. PMID: 34257452; PMCID: PMC9686310; Dalakas MC. Inflammatory Muscle Diseases. N Engl J Med. 2015 Jul 23;373(4):393-4. doi: 10.1056/NEJMc1506827. PMID: 26200989; Edouard P, Reurink G, Mackey AL, Lieber RL, Pizzari T, Järvinen TAH, Gronwald T, Hollander K. Traumatic muscle injury. Nat Rev Dis Primers. 2023 Oct 19;9(1):56. doi: 10.1038/s41572-023-00469-8. PMID: 37857686; Larsson L, Degens H, Li M, Salviati L, Lee Yl, Thompson W, Kirkland JL, Sandri M. Sarcopenia: Aging-Related Loss of Muscle Mass and Function. Physiol Rev. 2019 Jan 1;99(1):427-511. doi: 10.1152/physrev.00061.2017. PMID: 30427277; PMCID: PMC6442923; Morgan J, Partridge T. Skeletal muscle in health and disease. Dis Model Mech. 2020 Feb 6;13(2):dmm042192. doi: 10.1242/dmm.042192. PMID: 32066552; PMCID: PMC7044447).

### Subject

The subject can be an infant, a young child or a child, or older than a child like a teenager, an adult, including elderly.

The subject can be healthy or suffering or recovering from a disease, such as from a muscle disorder.

The subject can be older than a child, like a teenager, adult, including elderly.

The subject can be a sick infant, young child, child, older than child, like a teenager, adult, including elderly.

The subject can be a heatlhy infant, young child, child, older than child, like a teenager, adult, including elderly.

The subject can be a healthy infant, young child, child, older than child, like a teenager, adult, including elderly, recovering from a disease, such as from a muscle disorder.

The subject can be elderly with sarcopenia and/or frailty.

The subject is preferably an infant, a young child or a child.

The subject can be an infant, a young child, or a child born by C- section.

The subject can be an infant, a young child, or a child born preterm.

The subject can be an infant, a young child, or a child born small for gestational age (SGA). The subject can be an infant, a young child, or a child born with a low, very low, or extremely low weight at birth (LWB, VLBW, or ELBW).

The nutritional composition for use according to the invention may also be used in subject that was born by C-section or that was vaginally delivered.

In some embodiments, the subject was born by C- section and/or preterm, and/or small for gestational age (SGA) and/or with a low, very low, or extremely low weight at birth (LWB, VLBW, or ELBW) and/or experienced intra-uterine growth retardation (IUGR).

In some embodiments the nutritional composition for use according to the invention can be for use before and/or during the weaning period.

In some embodiments the nutritional composition for use according to the invention is for use in a subject at risk and/or in need.

The subject at risk and/or in need may be bottle-fed and/or formula-fed. The subject at risk and/or in need may be an infant, young child or child suffering from muscular dystrophy such as Duchenne disease, and/or an infant, young child or child born by C- section and/or preterm, and/or small for gestational age (SGA) and/or with a low, very low, or extremely low weight at birth (LWB, VLBW, or ELBW) and/or experienced intra-uterine growth retardation (IUGR), a stunted infant, young child or child, and/or a subject who has difficulties in standing-up for a certain time, grip object etc.. Suitably, a subject who has handgrip difficulties may be identified using the methods described herein for the assessment of handgrip.

In one embodiment the composition for use according to the invention is given to the subject as a supplementary composition to the mother's milk. In some embodiments the subject receives the mother's milk during at least the first 2 weeks, first 1, 2, 4, 6 or 12months. In one embodiment the nutritional composition for use according to the invention is given to the subject after such period of mother's nutrition or is given together with such period of mother's milk nutrition. In another embodiment the nutritional composition for use according to the invention is given to the subject as the sole or primary nutritional composition during at least one period of time, e.g. after the 1^{st}, 2^{nd} or 4^{th} month of life, during at least 1, 2, 4, 6 or 12 months. In one embodiment the nutritional composition of the invention is a complete nutritional composition (fulfilling all or most of the nutritional needs of the subject). In another embodiment the nutritional composition for use according to the invention is a supplement or a fortifier intended for example to supplement human milk or to supplement an infant formula or a follow-on formula.

The methods and uses in improving and/or increasing and/or maintaining and/or stimulating and/or promoting at least one muscle condition in a subject according to the present invention are therapeutic or non-therapeutic.

In some embodiments the methods and uses in improving and/or increasing and/or maintaining and/or stimulating and/or promoting at least one muscle condition in a subject according to the present invention are therapeutic.

In some embodiments the methods and uses in improving and/or increasing and/or maintaining and/or stimulating and/or promoting at least one muscle condition in a subject according to the present invention are non-therapeutic.

In some embodiments the nutritional composition is for use in treating at least one muscle condition in a subject.

In some embodiments the nutritional composition is for use in treating at least one muscle disorder in a subject.

In some embodiments the nutritional composition is for use in preventing at least one muscle disorder in a subject.

In some embodiments the nutritional composition is for use in improving and/or increasing at least one muscle condition in a subject, wherein the subject is infant, a young child or a child.

In some embodiments the nutritional composition is for use in stimulating at least one muscle condition in a subject, wherein the subject is infant, a young child or a child.

In some embodiments the nutritional composition is for use in improving and/or increasing at least one muscle condition in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).

In some embodiments the nutritional composition is for use in improving and/or increasing at least one muscle condition in a subject, wherein the subject is an infant, a young child or a child, or older than a child or a teenager, an adult, including elderly, recovering from a disease, notably from a muscle disorder.

In some embodiments the nutritional composition is for use in improving and/or increasing at least one muscle condition in a subject, wherein the subject is an infant, a young child or a child, or older than a child like a teenager, an adult, including elderly, suffering from a disease, notably from a muscle disorder.

In some embodiments the nutritional composition is for use in improving and/or increasing at least one muscle condition in a subject, wherein the subject is elderly, notably with sarcopenia and/or frailty.

In some embodiments the nutritional composition is for use in stimulating at least one muscle condition in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).

In some embodiments the nutritional composition is for use in stimulating at least one muscle condition in a subject, wherein the subject is an infant, a young child or a child, or older than a child or a teenager, an adult, including elderly, recovering from a disease, notably from a muscle disorder.

In some embodiments the nutritional composition is for use in stimulating at least one muscle condition in a subject, wherein the subject is an infant, a young child or a child, or older than a child like a teenager, an adult, including elderly, suffering from a disease, notably from a muscle disorder.

In some embodiments the nutritional composition is for use in stimulating at least one muscle condition in a subject, wherein the subject is elderly, notably with sarcopenia and/or frailty.

In some embodiments the nutritional composition is for use in promoting at least one muscle condition in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).

In some embodiments the nutritional composition is for use in promoting at least one muscle condition in a subject, wherein the subject is an infant, a young child or a child, or older than a child or a teenager, an adult, including elderly, recovering from a disease, notably from a muscle disorder.

In some embodiments the nutritional composition is for use in promoting at least one muscle condition in a subject, wherein the subject is an infant, a young child or a child, or older than a child like a teenager, an adult, including elderly, suffering from a disease, notably from a muscle disorder.

In some embodiments the nutritional composition is for use in promoting at least one muscle condition in a subject, wherein the subject is elderly, notably with sarcopenia and/or frailty.

In some embodiments the nutritional composition is for use in increasing muscle strength in a subject, wherein the subject is infant, a young child or a child.

In some embodiments the nutritional composition is for use in increasing muscle strength in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).

In some embodiments the nutritional composition is for use in increasing muscle strength in a subject, wherein the subject is an infant, a young child or a child, or older than a child or a teenager, an adult, including elderly, recovering from a disease, notably from a muscle disorder.

In some embodiments the nutritional composition is for use in increasing muscle strength in a subject, wherein the subject is an infant, a young child or a child, or older than a child like a teenager, an adult, including elderly, suffering from a disease, notably from a muscle disorder.

In some embodiments the nutritional composition is for use in increasing muscle strength in a subject, wherein the subject is elderly, notably with sarcopenia and/or frailty.

In some embodiments the nutritional composition is for use in increasing muscle force in a subject, wherein the subject is infant, a young child or a child.

In some embodiments the nutritional composition is for use in increasing muscle force in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).

In some embodiments the nutritional composition is for use in increasing muscle force in a subject, wherein the subject is an infant, a young child or a child, or older than a child or a teenager, an adult, including elderly, recovering from a disease, notably from a muscle disorder.

In some embodiments the nutritional composition is for use in increasing muscle force in a subject, wherein the subject is an infant, a young child or a child, or older than a child like a teenager, an adult, including elderly, suffering from a disease, notably from a muscle disorder.

In some embodiments the nutritional composition is for use in increasing muscle force in a subject, wherein the subject is elderly, notably with sarcopenia and/or frailty.

In some embodiments the nutritional composition is for use in increasing grip strength in a subject, wherein the subject is infant, a young child or a child.

In some embodiments the nutritional composition is for use in increasing grip strength in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).

In some embodiments the nutritional composition is for use in increasing grip strength in a subject, wherein the subject is an infant, a young child or a child, or older than a child or a teenager, an adult, including elderly, recovering from a disease, notably from a muscle disorder.

In some embodiments the nutritional composition is for use in increasing grip strength in a subject, wherein the subject is an infant, a young child or a child, or older than a child like a teenager, an adult, including elderly, suffering from a disease, notably from a muscle disorder.

In some embodiments the nutritional composition is for use in increasing grip strength in a subject, wherein the subject is elderly, notably with sarcopenia and/or frailty.

In some embodiments the nutritional composition is for use in improving endurance capacity in a subject, wherein the subject is infant, a young child or a child.

In some embodiments the nutritional composition is for use in increasing muscle quality in a subject, wherein the subject is infant, a young child or a child.

In some embodiments the nutritional composition is for use in increasing muscle quality in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).

In some embodiments the nutritional composition is for use in increasing muscle quality in a subject, wherein the subject is an infant, a young child or a child, or older than a child or a teenager, an adult, including elderly, recovering from a disease, notably from a muscle disorder.

In some embodiments the nutritional composition is for use in increasing muscle quality in a subject, wherein the subject is an infant, a young child or a child, or older than a child like a teenager, an adult, including elderly, suffering from a disease, notably from a muscle disorder.

In some embodiments the nutritional composition is for use in increasing muscle quality in a subject, wherein the subject is elderly, notably with sarcopenia and/or frailty.

In some embodiments the nutritional composition is for use in increasing muscle mass in a subject, wherein the subject is infant, a young child or a child.

In some embodiments the nutritional composition is for use in increasing muscle mass in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).

In some embodiments the nutritional composition is for use in increasing muscle mass in a subject, wherein the subject is an infant, a young child or a child, or older than a child or a teenager, an adult, including elderly, recovering from a disease, notably from a muscle disorder.

In some embodiments the nutritional composition is for use in increasing muscle mass in a subject, wherein the subject is an infant, a young child or a child, or older than a child like a teenager, an adult, including elderly, suffering from a disease, notably from a muscle disorder.

In some embodiments the nutritional composition is for use in increasing muscle mass in a subject, wherein the subject is elderly, notably with sarcopenia and/or frailty.

In some embodiments the nutritional composition is for use in increasing muscle specific force in a subject, wherein the subject is infant, a young child or a child.

In some embodiments the nutritional composition is for use in increasing muscle specific force in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).

In some embodiments the nutritional composition is for use in increasing muscle specific force in a subject, wherein the subject is an infant, a young child or a child, or older than a child or a teenager, an adult, including elderly, recovering from a disease, notably from a muscle disorder.

In some embodiments the nutritional composition is for use in increasing muscle specific force in a subject, wherein the subject is an infant, a young child or a child, or older than a child like a teenager, an adult, including elderly, suffering from a disease, notably from a muscle disorder.

In some embodiments the nutritional composition is for use in increasing muscle specific force in a subject, wherein the subject is elderly, notably with sarcopenia and/or frailty.

In some embodiments the nutritional composition is for use in modulating muscle health in a subject.

In some embodiments the nutritional composition is for use in increasing muscle force generation in a subject, wherein the subject is infant, a young child or a child.

In some embodiments the nutritional composition is for use in increasing muscle force generation in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).

In some embodiments the nutritional composition is for use in increasing muscle force generation in a subject, wherein the subject is an infant, a young child or a child, or older than a child or a teenager, an adult, including elderly, recovering from a disease, notably from a muscle disorder.

In some embodiments the nutritional composition is for use in increasing muscle force generation in a subject, wherein the subject is an infant, a young child or a child, or older than a child like a teenager, an adult, including elderly, suffering from a disease, notably from a muscle disorder.

In some embodiments the nutritional composition is for use in increasing muscle force generation in a subject, wherein the subject is elderly, notably with sarcopenia and/or frailty.

In some embodiments the nutritional composition is for use in promoting muscle health in a subject, wherein the subject is infant, a young child or a child.

In some embodiments the nutritional composition is for use in promoting muscle health in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).

In some embodiments the nutritional composition is for use in promoting muscle health in a subject, wherein the subject is an infant, a young child or a child, or older than a child or a teenager, an adult, including elderly, recovering from a disease, notably from a muscle disorder.

In some embodiments the nutritional composition is for use in promoting muscle health in a subject, wherein the subject is an infant, a young child or a child, or older than a child like a teenager, an adult, including elderly, suffering from a disease, notably from a muscle disorder.

In some embodiments the nutritional composition is for use in promoting muscle health in a subject, wherein the subject is elderly, notably with sarcopenia and/or frailty.

In some embodiments the nutritional composition is for use in promoting normal muscle function in a subject.

In some embodiments the nutritional composition is for use in modulating muscle stem cells function in a subject.

In some embodiments the nutritional composition is for use in promoting and/or increasing muscle stem cells function in a subject, wherein the subject is infant, a young child or a child.

In some embodiments the nutritional composition is for use in promoting and/or increasing muscle stem cells function in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).

In some embodiments the nutritional composition is for use in promoting and/or increasing muscle stem cells function in a subject, wherein the subject is an infant, a young child or a child, or older than a child or a teenager, an adult, including elderly, recovering from a disease, notably from a muscle disorder.

In some embodiments the nutritional composition is for use in promoting and/or increasing muscle stem cells function in a subject, wherein the subject is an infant, a young child or a child, or older than a child like a teenager, an adult, including elderly, suffering from a disease, notably from a muscle disorder.

In some embodiments the nutritional composition is for use in promoting and/or increasing muscle stem cells function in a subject, wherein the subject is elderly, notably with sarcopenia and/or frailty.

In some embodiments the invention concerns the non-therapeutic use of the nutritional composition for improving and/or increasing and/or maintaining and/or stimulating and/or promoting and/or preventing at least one muscle condition in a subject, such as infant, a young child or a child, selected form the list consisting of:
increasing muscle strength;
increasing muscle force;
increasing grip strength;
improving endurance capacity;
increasing muscle quality;
increasing or maintaining muscle mass;
increasing muscle specific force;
increasing exercise capacity;
increasing muscle force generation;
promoting normal muscle function;
modulating muscle stem cells function;
promoting muscle stem cell function;
increasing muscle stem cell function;
and any combinations thereof.

In some embodiments the invention concerns the non-therapeutic use of the nutritional composition for improving and/or increasing and/or maintaining and/or stimulating and/or promoting and/or preventing at least one muscle condition in a subject, such as infant, a young child or a child, selected form the list consisting of:
increasing muscle strength;
increasing muscle force;
increasing grip strength;
and any combinations thereof.

In some embodiments the invention concerns the non-therapeutic use of the nutritional composition for improving at least one muscle condition in a subject, notably a healthy subject, like a healthy adult.

In some embodiments the invention concerns the non-therapeutic use of the nutritional composition for increasing at least one muscle condition in a subject, notably a healthy subject, like a healthy adult.

In some embodiments the invention concerns the non-therapeutic use of the nutritional composition for maintaining at least one muscle condition in a subject, notably a healthy subject, like a healthy adult.

In some embodiments the invention concerns the non-therapeutic use of the nutritional composition for stimulating at least one muscle condition in a subject, notably a healthy subject, like a healthy adult.

In some embodiments the invention concerns the non-therapeutic use of the nutritional composition for promoting at least one muscle condition in a subject, notably a healthy subject, like a healthy adult.

In some embodiments the invention concerns the non-therapeutic use of the nutritional composition for improving endurance capacity in a subject, notably a healthy subject, like a healthy adult.

In some embodiments the invention concerns the non-therapeutic use of the nutritional composition for maintaining muscle mass in a subject, notably a healthy subject, like a healthy adult.

In some embodiments the invention concerns the non-therapeutic use of the nutritional composition for increasing exercise capacity in a subject, notably a healthy subject, like a healthy adult.

In some embodiments the invention concerns the non-therapeutic use of the nutritional composition for modulating muscle health in a subject, notably a healthy subject, like a healthy adult.

In some embodiments the invention concerns the non-therapeutic use of the nutritional composition for promoting normal muscle function in a subject, notably a healthy subject, like a healthy adult.

In some embodiments the invention concerns the non-therapeutic use of the nutritional composition for modulating muscle stem cells function in a subject, notably a healthy subject, like a healthy adult.

### Other ingredients

The nutritional composition according to the present invention may also comprise other types of oligosaccharide(s), polysaccharides and/or a fiber(s) and/or a precursor(s) thereof. The other oligosaccharide and/or fiber and/or precursor thereof may be selected from the list comprising human milk oligosaccharides (HMOs), galacto-oligosaccharides (GOS), fructo-oligosaccharides (FOS), xylooligosaccharides (XOS), cello-oligosaccharides (COS), arabinoxylans, arabinans, xylans, inulin, polydextrose, beta-glucans, pectins and any combination thereof and any derived products, like partial hydrolysis products, thereof. They may be in an amount between 0 and 10% by weight of composition. In a particular embodiment, the nutritional composition or the combination can also contain at least one BMO (bovine milk oligosaccharide).

HMOs which may be included in the nutritional composition or combination according to the present invention may be selected from the group consisting of 2'-FL (2'- fucosyllactose), 3-FL (3- fucosyllactose), Lacto-difucotetraose (LDFT)), lacto-N-fucopentaose I, lacto-N-fucopentaose II, lacto-N- fucopentaose III, lacto-N-fucopentaose V, lacto-N-fucohexaose, lacto-N-difucohexaose I (LNDFH I), fucosyllacto-N-hexaose, fucosyllacto-N-neohexaose, difucosyllacto-N-hexaose I, difucosyllacto-N-neohexaose II, para-lacto-N-neohexaose (para-LNnH), LNT (lacto-N-tetraose), LNnT (lacto-N-neotetraose), lacto-N-hexaose, lacto- N-neohexaose, para-lacto-N-hexaose, para-lacto-N-neohexaose, lacto-N-octaose, lacto-N-neooctaose, iso- lacto-N-octaose, para- lacto-N-octaose, lacto-N-decaose, 3'-SL (3' sialyllactose), 6'-SL (6' sialyllactose), A-tetrasaccharide and any combination thereof.

In some embodiments, the nutritional composition according to the invention comprises at least one additional HMO. In other embodiments, the nutritional composition according to the present invention is devoid of any further HMOs.

The nutritional composition according to the invention generally contains a protein source. The protein can be in an amount of from 1.6 to 3 g per 100 kcal. In some embodiments, the protein amount can be between 2.4 and 4 g/100kcal or more than 3.6 g/100kcal. In some other embodiments the protein amount can be below 2.0 g per 100 kcal, e.g. between 1.8 to 2 g/100 kcal, or in an amount below 1.8 g per 100 kcal.

The protein sources are not particularly limited, they can be based on whey (native or not), casein and mixtures thereof may be used as well as protein sources based on soy. As far as whey proteins are concerned, the protein source may be based on acid whey or sweet whey or mixtures thereof and may include alpha-lactalbumin and beta-lactoglobulin in any desired proportions.

In some advantageous embodiments, the protein source is whey predominant (i.e. more than 50% of proteins are coming from whey proteins, such as 60% or 70%).

The proteins may be intact or hydrolysed or a mixture of intact and hydrolysed proteins. By the term "intact" is meant that the main part of the proteins are intact, i.e. the molecular structure is not altered, for example at least 80% of the proteins are not altered, such as at least 85% of the proteins are not altered, preferably at least 90% of the proteins are not altered, even more preferably at least 95% of the proteins are not altered, such as at least 98% of the proteins are not altered. In a particular embodiment, 100% of the proteins are not altered.

The term "hydrolysed" means in the context of the present invention a protein which has been hydrolysed or broken down into its component amino acids. The proteins may be either fully or partially hydrolysed. It may be desirable to supply partially hydrolysed proteins (degree of hydrolysis between 2 and 20%), for example for infants or young children believed to be at risk of developing cow's milk allergy. If hydrolysed proteins are required, the hydrolysis process may be carried out as desired and as is known in the art. For example, whey protein hydrolysates may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

In an embodiment of the invention, at least 70% of the proteins are hydrolysed, preferably at least 80% of the proteins are hydrolysed, such as at least 85% of the proteins are hydrolysed, even more preferably at least 90% of the proteins are hydrolysed, such as at least 95% of the proteins are hydrolysed, particularly at least 98% of the proteins are hydrolysed. In a particular embodiment, 100% of the proteins are hydrolysed.

In one particular embodiment, the proteins of the nutritional composition are hydrolysed, fully hydrolysed or partially hydrolysed. The degree of hydrolysis (DH) of the protein can be between 8 and 40, or between 20 and 60 or between 20 and 80 or more than 10, 20, 40, 60, 80 or 90.

The protein component can alternatively be replaced by a mixture or synthetic amino acid.

The nutritional composition according to the present invention generally contains a carbohydrate source. This is particularly preferable in the case where the nutritional composition of the invention is an infant formula. In this case, any carbohydrate source conventionally found in infant formulae such as lactose, sucrose, saccharose, maltodextrin, starch and mixtures thereof may be used although one of the preferred sources of carbohydrates is lactose.

The nutritional composition according to the present invention generally contains a source of lipids. This is particularly relevant if the nutritional composition of the invention is an infant formula. In this case, the lipid source may be any lipid or fat which is suitable for use in infant formulae. Some suitable fat sources include palm oil, structured triglyceride oil, high oleic sunflower oil and high oleic safflower oil, medium-chain-triglyceride oil. The essential fatty acids linoleic and α-linolenic acid may also be added, as well small amounts of oils containing high quantities of preformed arachidonic acid and docosahexaenoic acid such as fish oils or microbial oils. The fat source may have a ratio of n-6 to n-3 fatty acids of about 5:1 to about 15:1; for example about 8:1 to about 10:1.

In some embodiments, in said lipids comprising fatty acid (FA) triacylglycerols (TAG), said fatty acid TAG comprise high oleic sunflower oil.

In some embodiments, in said lipids comprising fatty acid (FA) triacylglycerols (TAG), said fatty acid TAG comprise stearic, palmitic and myristic esters, wherein the sum of the amount of stearic, palmitic and myristic esters at the sn-1(3) position of the TAG is less than 13.0 wt-% of TAG such as of from 4.0 wt-% of TAG to 12.9 wt-% of TAG or of form 4.0 wt-% of TAG to 11.0 wt-% of TAG;
and the myristic acid represents up to 3.7 wt-% of the total FA, such as of from 2.2 wt-% of the total FA to 3.7 wt-% of the total FA or of from 2.5 wt-% of the total FA to 3.1 wt-% of the total FA; and
the palmitic acid represents up to 11.1 wt-% of the total FA, such as of from 3.0 wt-% of the total FA to 11.0 wt-% of the total FA or of from 3.5 wt-% of the total FA to 6.1 wt-% of the total FA.

In some embodiments of the lipids, stearic acid represents up to 4.6 wt-% of the total FA, such as of from 2.2 wt-% of the total FA to 4.5 wt-% of the total FA or of from 3.0 wt-% of the total FA to 3.7 wt-% of the total FA.

In some embodiments of the lipids, the SCFA (short chain fatty acid) are present in less than 40 wt-%, such as of form 10 wt-% to 35 wt-% or of form 10 wt-% to 23 wt-%, and the MUFA (mono-unsaturated fatty acid) are present more than 35.1 %, such as of form 35.2 wt-% to 64.5 wt-% or of form 50.0 wt-% to 60.0 wt-%.

In some embodiments, said lipids comprise a mixture of oils selected from: 16.9 to 22.0% of rapeseed oil, 11.5 to 14.5% of sunflower oil, 40.0 to 58% of high oleic sunflower oil, and 15.0% to 20.5% of coconut oil.

The nutritional composition of the invention may also contain all vitamins and minerals understood to be essential in the daily diet and in nutritionally significant amounts. Minimum requirements have been established for certain vitamins and minerals. Examples of minerals, vitamins and other nutrients optionally present in the composition of the invention include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chlorine, potassium, sodium, selenium, chromium, molybdenum, taurine, and L-carnitine. Minerals are usually added in salt form. The presence and amounts of specific minerals and other vitamins will vary advantageously according to the invention.

If necessary, the nutritional composition of the invention may contain emulsifiers and stabilisers such as soy, lecithin, citric acid esters of mono- and di-glycerides, and the like.

The nutritional composition of the invention may also contain other substances which may have a beneficial effect such as lactoferrin, nucleotides, nucleosides, and the like.

The nutritional composition of the invention may also contain carotenoid(s). In some particular embodiments of the invention, the nutritional composition of the invention does not comprise any carotenoid.

In some particular embodiments, the nutritional composition of the invention may also contain at least one further probiotic besides pre-conditioned *L. reuteri.*

The further probiotic is a probiotic bacterial strain. In some specific embodiments, it is particularly Bifidobacteria and/or Lactobacilli.

Suitable probiotic bacterial strains include *Lactobacillus rhamnosus* ATCC 53103 available from Valio Oy of Finland under the trademark LGG, *Lactobacillus rhamnosus* CGMCC 1.3724, *Lactobacillus paracasei* CNCM I-2116, *Lactobacillus johnsonii* CNCM I-1225, *Streptococcus salivarius* DSM 13084 sold by BLIS Technologies Limited of New Zealand under the designation KI2, *Bifidobacterium lactis* CNCM 1-3446 sold inter alia by the Christian Hansen company of Denmark under the trademark Bb 12, *B. longum* CNCM I-2618 (B. longum NCC2705), *Bifidobacterium breve* sold by Danisco under the trademark Bb-03, Bifidobacterium breve sold by Morinaga under the trade mark M-16V, *Bifidobacterium infantis* sold for example by Procter & Gamble Co. under the trademark Bifantis and *Bifidobacterium breve* sold by Institut Rosell (Lallemand) under the trademark R0070, *Bifidobacterium dentium sold by Creative Biolabs, Bifidobacterium longum subsp. Infantis* LMG 11588 (also known as ATCC 17930), one or more member of the genus *Dorea* such as *dorea phocaeensis, Dorea formicigenerans* (ATCC 27755), *B. kashiwanohense* (JCM 15439) and/or B. *kashiwanohense* (DSM 21854) and *Limosilactobacillus* at the genus level.

In another aspect, the invention concerns *Bifidobacterium dentium* and/or *Dorea formicigenerans* and/or *dorea phocaeensis* and nutritional compositions comprising *Bifidobacterium dentium* and/or *Dorea formicigenerans* and/or *dorea phocaeensis (phocaensis)* for use in improving and/or stimulating and/or promoting muscle condition in a subject, wherein the muscle condition, *Bifidobacterium dentium* and/or *Dorea formicigenerans* and/or *dorea phocaeensis,* subjects and nutritional compositions are preferably as defined in this patent application.

The nutritional composition or combination according to the invention may contain from 10³ to 10¹² cfu of the at least one (further) probiotic strain, more preferably between 10⁷ and 10¹² cfu such as between 10⁸ and 10¹⁰ cfu of probiotic strain per g of composition on a dry weight basis.

In one embodiment, the probiotics are viable. In another embodiment, the probiotics are non-replicating or inactivated. There may be both viable probiotics and inactivated probiotics in some other embodiments. Probiotic components and metabolites can also be added.

*B. kashiwanohense* was isolated from healthy infant faeces. For example, this bacterium has previously been characterized by determining its phenotypic and biochemical features and phylogenetic positions based on partial 16S rRNA gene sequence analysis (Morita et al., International Journal of Systematic and Evolutionary Microbiology, 2011, 61: 2610-2615). The GenBank/EMBL/DDBJ accession numbers for the 16S rRNA and partial hsp60 gene sequences of two strains of B. *kashiwanohense* are (i) AB491757 and AB578933 and (ii) are AB425276.2 and AB491759.2, respectively. One strain of B. *kashiwanohense* is publicly available from two collections with the accession numbers JCM 15439 and DSM 21854 (Morita et al., International Journal of Systematic and Evolutionary Microbiology, 2011, 61: 2610-2615).

The B. *kashiwanohense* may be a B. *kashiwanohense* having at least 99% (suitably, at least 99.9%) Average Nucleotide Identity (ANI) to any *B. kashiwanohense* known to the skilled person.

In some embodiments, *dorea phocaeensis* strain is strain Marseille-P4003^{T} exhibiting a 98.23% sequence identity with *Dorea formicigenerans* strain ATCC 27755 (GenBank accession number NR044645), (Takakura et al., Dorea phocaeensis sp. nov., a new bacterium isolated from the stool of a healthy 29-year-old male. New Microbes and New Infections, Volume 32, November 2019, 100600.)

As used herein the term "Average nucleotide identity (ANI)" refers to a distance-based approach to delineate species based on pair-wise comparisons of their genome sequences. ANI is an in silico approach for phylogenetic definition of a species and has become the gold standard for species delineation (Goris et al., 2007, Int. J. Syst. Evol. Microbiol. 57: 81-91; Kim et al., 2014, Int. J. Syst. Evol. Micr. 64: 346-351; Richter et al., 2009, P Natl Acad Sci USA 106: 19126-19131; and Chan et al., 2012, Bmc. Microbiol. 12).

The ANI of the shared genes between two strains is known to be a robust means to compare genetic relatedness among strains. Strains with ANI values of at least about 96% can be considered to belong to the same species (Konstantinidis and Tiedje, 2005, Proc Natl Acad Sci USA, 102(7):2567-72; and Goris et al., 2007, Int Syst Evol Microbiol. 57(Pt 1):81-91), while ANI values of at least about 99% indicate that the bacterial genomes belong to the same strain. The ANI between two bacterial genomes is calculated from pair-wise comparisons of all sequences shared between any two strains and can be determined, for example, using any of a number of publicly available ANI tools, including but not limited to OrthoANI with usearch (Yoon et al., 2017, Antonie van Leeuwenhoek 110:1281-1286); ANI Calculator, JSpecies (Richter and Rossello-Mora, 2009, Proc Natl Acad Sci USA 106:19126-19131); and JSpeciesWS (Richter et al., 2016, Bioinformatics 32:929-931). Other methods for determining the ANI of two genomes are known in the art (Konstantinidis, K. T. and Tiedje, 2005, J. M., Proc. Natl. Acad. Sci. U.S.A., 102: 2567-2572; and Varghese et al., 2015, Nucleic Acids Research, 43(14):6761-6771).

Suitably, the B. *kashiwanohense* has at least 99% (suitably, at least 99.1%, at least 99.2%, at least 99.3%, at least 99.4%, at least 99.5%, at least 99.6%, at least 99.7%, at least 99.8%, at least 99.9%) ANI to B. *kashiwanohense* JCM 15439 and/or B. *kashiwanohense* DSM 21854. Preferably, the B. *kashiwanohense* has at least 99.9% ANI to B. *kashiwanohense* JCM 15439 and/or B. *kashiwanohense* DSM 21854.

### Nutritional composition

The nutritional composition according to the invention can be for example an infant formula, a starter infant formula, a follow-on or follow-up formula, a growing-up milk, a baby food (such as yogurt, pureed or mashed baby food), an infant cereal composition, a fortifier such as a human milk fortifier or a supplement. In some particular embodiments, the composition of the invention is an infant formula, a fortifier or a supplement that may be intended for the first 4 or 6 months of age. In a preferred embodiment the nutritional composition of the invention is an infant formula.

The composition according to the invention can be for example formulated as an infant formula, a starter infant formula, a follow-on or follow-up formula, a growing-up milk, a baby food (such as yogurt, pureed or mashed baby food), an infant cereal composition, a fortifier such as a human milk fortifier or a supplement.

In some other embodiments the nutritional composition for use according to the present invention is a fortifier. The fortifier can be a breast milk fortifier (e.g. a human milk fortifier) or a formula fortifier such as an infant formula fortifier or a follow-on/follow-up formula fortifier.

When the nutritional composition for use according to the invention is a supplement, it can be provided in the form of unit doses. In such cases it is particularly useful to define the amount of oligosaccharides in terms of daily dose to be administered to the infant, young child or child.

Suitably, when the nutritional composition for use according to the invention is a supplement, it may comprise pre-conditioned *L. reuteri* and no other additional nutrient on top of the excipients necessary to obtain a stable composition. Suitably, when the nutritional composition for use according to the invention is a supplement, it may comprise *L. reuteri* in combination with one or more further HMOs (e.g. one or more further HMOs as described herein), and no other additional nutrient on top of the excipients necessary to obtain a stable composition. Suitably, when the nutritional composition for use according to the invention is a supplement, it may comprise *L. reuteri* in combination with one or more vitamins and/or minerals, and no other additional nutrient on top of the excipients necessary to obtain a stable composition.

The nutritional composition for use according to the invention can be in solid (e.g. powder), liquid or gelatinous form. In a specific embodiment the nutritional composition for use according to the invention is a supplement, wherein the supplement is in powder form and provided in a sachet, preferably a sachet with 0.1 to 20 g per sachet, for example 1 to 10 g per sachet, or in the form of a syrup, preferably a syrup with a total solid concentration of 5 to 75 g/100 mL (5 to 75% (w/v)). When the supplement is in powder form, it may comprise a carrier. It is however preferred that the supplement is devoid of a carrier. When the supplement is in the form of a syrup, the components are preferably dissolved or suspended in water acidified with citrate.

In a particular embodiment the nutritional composition for use according to the invention according to the invention is a hypoallergenic composition. In another particular embodiment the composition for use according to the invention according to the invention is a hypoallergenic nutritional composition.

The nutritional composition for use according to the invention according to the invention may be prepared in any suitable manner. A composition will now be described by way of example.

For example, a formula such as an infant formula may be prepared by blending together the protein source, the carbohydrate source and the fat source in appropriate proportions. If used, the emulsifiers may be included at this point. The vitamins and minerals may be added at this point but they are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The temperature of the water is conveniently in the range between about 50°C and about 80°C to aid dispersal of the ingredients. Commercially available liquefiers may be used to form the liquid mixture.

The oligosaccharide(s) may be added at this stage, especially if the final product is to have a liquid form. If the final product is to be a powder, they may likewise be added at this stage if desired.

The liquid mixture is then homogenised, for example in two stages.

The liquid mixture may then be thermally treated to reduce bacterial loads, by rapidly heating the liquid mixture to a temperature in the range between about 80°C and about 150°C for a duration between about 5 seconds and about 5 minutes, for example. This may be carried out by means of steam injection, an autoclave or a heat exchanger, for example a plate heat exchanger.

Then, the liquid mixture may be cooled to between about 60°C and about 85°C for example by flash cooling. The liquid mixture may then be again homogenised, for example in two stages between about 10 MPa and about 30 MPa in the first stage and between about 2 MPa and about 10 MPa in the second stage. The homogenised mixture may then be further cooled to add any heat sensitive components, such as vitamins. The pH and solids content of the homogenised mixture are conveniently adjusted at this point.

If the final product is to be a powder, the homogenised mixture is transferred to a suitable drying apparatus such as a spray dryer or freeze dryer and converted to powder. The powder should have a moisture content of less than about 5% by weight. The oligosaccharide(s) may also or alternatively be added at this stage by dry-mixing or by blending them in a syrup form of crystals, along with the probiotic strain, and the mixture is spray-dried or freeze-dried.

If a liquid composition is preferred, the homogenised mixture may be sterilised then aseptically filled into suitable containers or may be first filled into the containers and then retorted.

In another embodiment, the composition or combination of the invention may be a supplement. The supplement may be in the form of tablets, capsules, pastilles or a liquid for example. When the supplement is in the form of a liquid, it can be an aqueous solution containing 1-50 wt-% HMO, preferably 5-35 wt-% HMO, more preferably 5-15 wt-%, such as 10 wt-% HMO. The supplement in the form of a liquid can be a tea or a juice.

The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents and gel forming agents. The supplement may also contain conventional pharmaceutical additives and adjuvants, excipients and diluents, including, but not limited to, water, gelatine of any origin, vegetable gums, lignin-sulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like.

Further, the supplement may contain an organic or inorganic carrier material suitable for oral or parenteral administration as well as vitamins, minerals trace elements and other micronutrients in accordance with the recommendations of Government bodies such as the USRDA.

The different embodiments, details and examples previously described in the specification (e.g. related to the types and amounts of oligosaccharide, the nutritional composition, the administration, the targeted population...) also apply to all these other objects.

### Vitamins

The forms (vitamers) of the vitamins used according to the invention is not particularly limited. The term vitamin as used herein refers to the vitamin and /or a metabolite thereof.

For example, as used herein, "vitamin B6" can include one or more of the following: pyridoxine (PN), pyridoxal 5'-phosphate (PLP), pyridoxine 5'-phosphate (P5P), pyridoxal (PL), pyridoxamine (PM), pyridoxamine 5 '-phosphate (PMP), 4-pyridoxic acid, and pyritinol. In a preferred embodiment, at least a portion of any vitamin B6 is PN. At least a portion of the vitamin B6 can be PLP.

Nicotinamide, also known as niacinamide or nicotinic acid amide, is the water-soluble, active form of vitamin B3. The term vitamin B3, also colloquially referred to as niacin, encompasses three vitamers: niacin (nicotinic acid), nicotinamide, and nicotinamide riboside.

Vitamin B9 can be as naturally-occurring (folate) or as its synthetic form (folic acid), see Hwang et al.; Arch. Pharm. Res. (2019) 42:319-325. In some embodiments vitamin B9 is folic acid.

In one embodiment, the vitamin D or metabolite thereof is vitamin D3 (cholecalciferol), vitamin D2 (ergocalciferol), 25- hydroxyvitamin D (calcidiol) and/or 1,25-dihydroxyvitamin D (calcitriol). In one embodiment, the vitamin D or metabolite thereof is vitamin D3. In one embodiment, the vitamin D or metabolite thereof is vitamin D2. In one embodiment, the vitamin D or metabolite thereof is 25-hydroxyvitamin D. In one embodiment, the vitamin D or metabolite thereof is 1,25-dihydroxyvitamin D. In a preferred embodiment, the vitamin D or metabolite thereof is 25-hydroxyvitamin D. In a preferred embodiment, the vitamin D or metabolite thereof is 25-hydroxyvitamin D and 1,25- dihydroxyvitamin D. Even more particularly the vitamin D3 is Cholecalciferol.

Vitamin B7 also known as biotin plays an important role in energy metabolism in muscle by converting carbohydrates, fats and proteins into energy and synthesizing glucose.

Vitamin C also known as ascorbic acid plays a role in reducing muscle fatigue. It acts as an antioxidant to metabolise carbohydrates and protect against exercise-induced oxidative stress as well as helping to absorb iron. It also helps to in the synthesis of collagen which is needed in connective tissue repair. Ascorbic acid is the only chemically active form of vitamin C. However, there are several different forms of vitamin C that include sodium ascorbate, calcium ascorbate, magnesium ascorbate.

Vitamin B12 is also known as cobalamin. Vitamin B12 is one of the B-vitamins and is soluble in water. There are different forms of vitamin B12, including Cyanocobalamin, Methylcobalamin, Adenosylcobalamin, and Hydroxocobalamin.

Vitamin E also known as alpha-tocopherol is the most biologically active form of vitamin E. Other forms of vitamin E include beta-tocopherol, gamma-tocopherol and delta-tocopherol. Vitamin E plays a crucial role in maintaining the health of cells and protecting them from damage caused by free radicals.

### L. reuteri

A *L. reuteri* strain as used throughout the current invention may generally be any *L. reuteri* strain, more preferably any commercially available *L. reuteri* strain. In this context, the terms *"L. reuteri*", *"L. reuteri* strain", and *"L. reuteri* bacteria" may be used synonymously. It is noted thereby that in the last decades DNA analysis tools have become more sophisticated, which has enabled scientists to discover many new bacterial species as well as realizing that the species historically grouped under the *Lactobacillus* genus were too diverse and did not conform to nomenclature conventions. To keep the probiotic groups accurate and organized, the genus *Lactobacillus* was therefore split into 25 different genera, including 23 novel genera. As a result, many probiotics have recently been given new genus names. Therefore, an alternative genus name for *Lactobacillus reuteri (L. reuteri)* is *Limosilactobacillus reuteri.* Since the reclassification process was relatively recent, we will use the former genera nomenclature, i.e., *Lactobacillus* and the new one, i.e., *Limosilactobacillus* interchangeably in this application.

Currently preferred bacteria provided in the compositions are bacteria of at least one *L. reuteri* strain or of multiple, i.e., at least two, *L. reuteri* strains.

A currently preferred *L. reuteri* strain is *L. reuteri* DSM 17938.

*Lactobacillus reuteri* DSM 17938 was deposited under the Budapest Treaty at the DSMZ-Deutsche Sammlung von Mikroorganismen und Zellkulturen GmbH (Mascheroder Weg 1 b, D-38124 Braunschweig, Germany) on January 30, 2006. The accessibility was further demonstrated on filing of WO2022/191767 A1.

The term "GOS" as used herein means "galacto-oligosaccharide". Galacto-oligosaccharides (GOS) as used herein typically consist of b-linked galactose moieties with galactose or glucose at the reducing end. Such GOS contains β-(1→2), β-(1→3), β-(1→4), or β-(1→6) linked galactose moieties and may have a degree of polymerization (DP) of 3-8 galactose units. The term GOS is therefore preferably referred to as oligosaccharide(s) comprising at least three galactose units, more preferably as oligosaccharide(s) comprising at least four galactose units, preferably having a degree of polymerization (DP) of 3-8 galactose units. Oligosaccharides occur naturally in the milk of some mammals, e.g., cow and human. GOS are commercially available and can be synthesized via biosynthesis processes from lactose by trans-galactosidase activity of b-galactosidases. GOS formation in biosynthesis procedures is usually favored by high concentrations of lactose or lactulose, incomplete lactose turnover, low water activity, and the use of enzymes with preference for trans-galactosylation. The linkage type(s) of resulting GOS from such processes is(are) specific for the enzymes used for biosynthesis.

One particular type of mixture of carbohydrates that can advantageously be used herein as a source of GOS to grow the probiotic bacteria is a mixture of GOS and of cow's milk oligosaccharides. Inparticular mixtures of GOS with 3'-sialyllactose (3'-SL) and/or 6'-sialyllactose (6'-SL) are preferably used. Indeed, such mixtures contain some oligosaccharides similar to human milk, which is particularly advantageous when the composition of the invention is used as an infant formula or as a nutritional supplement for infants. Such advantageous effects are described for example in Simeoni et al.; "Gut microbiota analysis reveals a marked shift to bifidobacteria by a starter infant formula containing a synbiotic of bovine milk-derived oligosaccharides and Bifidobacterium animalis subsp. lactis CNCM I- 3446"; Environ Microbiol, 2016, 18(7): 2185-2195. Such compositions can typically be obtained from concentrating whey permeate to obtain a concentrated bovine milk oligosaccharide composition and either adding GOS or generating the GOS in situ from hydrolysis of lactose by the action of a b- galactosidase.

The GOS source used in step a) of the inventive method to pre-condition the probiotic *L. reuteri* strain and the GOS source further added in step b) for preparing a final composition comprising the preconditioned probiotic *L. reuteri* strain with GOS, can be provided in the form of essentially pure GOS (i.e., ingredient having at least 90% GOS) or as part of a mixture, such as a mixture of carbohydrates, comprising GOS.

One aspect for the pre-conditioning of a probiotic *L. reuteri* strain with GOS is that a sufficient amount of GOS is provided in the growth medium. According to one embodiment, the GOS source is therefore typically added to the growth medium in step a) of the inventive method for preparing a probiotic composition in an amount such as providing at least 0.2 wt-%, preferably at least 0.5 wt-%, even more preferably at least 0.75 wt-% of GOS in the growth medium. In a preferred embodiment, GOS is provided in an amount such as providing at most 8 wt-% of GOS, such as about 7 wt-% GOS, 6 wt-% GOS, or even 5 wt-% GOS at most, more preferably at most 4 wt-% of GOS or even at most 3 wt-% GOS, in the growth medium, preferably calculated on basis of the growth medium (% w/w). Any combination of these upper and lower ranges is encompassed herewith. Preferably, GOS is added to the growth medium in a range of 0.2 wt-% to 8 wt-% or even 0.2 wt-% to 7 wt-%, or 0.2 wt-% to 6 wt-%, such as in a range of 0.5 wt-% to 8 wt-% or even 0.5 wt-% to 7 wt-%, or 0.5 wt-% to 6 wt-%, or in a range of 0.75 wt-% to 8 wt-% or even 0.75 wt-% to 7 wt-%, or 0.75 wt-% to 6 wt-%. Preferably, GOS is added to the growth medium in a range of 0.2 wt-% to 8 wt-%, preferably in a range of 0.5 wt % to 7 wt-%, more preferably in a range of 0.75 wt-% to 6 wt-%, and most preferably in a range of 0.75 wt-% to 5 wt-%, such as a range of 0.75 wt-% to 3 wt-% or 0.75 wt-% to 4 wt-%, preferably calculated on basis of the growth medium.

When a mixture of carbohydrates is used as a source of GOS for pre-conditioning a probiotic *L. reuteri* strain or when preparing the final composition containing the pre-conditioned probiotic *L. reuteri* strain with GOS, it is preferred that the amount of GOS in such a mixture is at least 20 wt-%, preferably at least 30 wt-%, more preferably at least 40 wt-%, even more preferably at least 45 wt-%, most preferably at least 48 wt-%, preferably calculated on a dry weight basis of the mixture containing GOS.

In an embodiment, GOS is used use as substrate, such as fermentation substrate, for the probiotic *L. reuteri* strain in step a). Hence, in such an embodiment, GOS is used as a carbon source during cultivation or fermentation of the probiotic *L. reuteri* strain.

Although it is not necessary that the bacteria consume only GOS as carbon source during the fermentation, high proportion of GOS in the GOS source during the pre-conditioning step favors the consumption of GOS by the bacteria during fermentation over consumption of other carbohydrates, leading to improved pre-conditioning effect. Smaller carbohydrates such as di-saccharides (lactose for instance) can also be present and be consumed by the bacteria supporting the pre-conditioning effect. Accordingly, it is also preferred that during the pre-conditioning step ai) the GOS source comprises at most 55 wt-%, preferably at most 50 wt-%, more preferably at most 45 wt-%, most preferably at most 42 wt-% of mono- or di-saccharides and/or no more than 40 wt-%, preferably no more than 30 wt-% lactose, preferably calculated on a dry weight basis of the mixture containing GOS. As an example, a mixture of carbohydrate used as a source of GOS during the pre-conditioning step may comprise GOS in amounts defined above for the mixture and the remainder formed by lactose, glucose, and/or galactose, and optionally 3'-SL and/or 6'-SL.

The prebiotic GOS may be added to the pre-conditioned probiotic *L. reuteri* strain any time following culturing in step ai). If the method comprises additional steps as mentioned above, i.e., washing and/or drying, GOS could be added prior to, during and/or following any such steps. In a particular embodiment, GOS is added to the pre-conditioned probiotic *L. reuteri* strain following the drying step.

GOS could be added to the pre-conditioned probiotic *L. reuteri* strain once or at multiple different occasions during the method.

According to a preferred embodiment, GOS may be added in step b) to the pre-conditioned probiotic *L. reuteri* strain obtained from step a) in an amount of at least 1 or 2 g, preferably at least 3 g per, likewise preferably between 2 to 12 g per, or between 2 to 10 g per, between 2 to 8 g per, or between 2 to 7 g, more preferably between 3 to 12 g, likewise more preferably between 3 and 10 g, such as between 3 to 8 g, between 3 to 7 g, between 3 to 6 g, or about 2, 3, 4, 5, 6, 7, 8, 9, 10, 11, or 12 g or any range formed thereby, to prepare the (final) probiotic composition. The above mentioned amounts of GOS added in step b) are preferably amount of GOS per daily dose. Hence, in a preferred embodiment, the probiotic composition is provided in a dosage form or unit, which is further described herein. In such a case, such a dosage form or unit preferably comprises the above-mentioned amount of GOS. For instance, a dosage unit of the probiotic composition comprises at least 1 g GOS. This means that step b) then corresponds to adding GOS to the pre-conditioned probiotic *L. reuteri* strain obtained from step aii) to prepare a dosage unit of the probiotic composition and where this dosage unit comprises at least 1 g GOS.

In some embodiments, an additional effective amount of GOS is added to the composition comprising an effective amount of a pre-conditioned probiotic *L. reuteri strain,* wherein the pre-conditioned probiotic L. *reuteri* strain has been prepared by cultivating a *L. reuteri* strain in the presence of GOS in a growth medium.

In some embodiments, the additional effective amount of GOS added to the composition comprising an effective amount of a pre-conditioned probiotic *L. reuteri strain* is added before the addition of further micro- and macronutrient.

The nutritional composition comprises an effective amount of a harvested, pre-conditioned L. *reuteri* strain and an effective amount of GOS added or supplemented, i.e., in the form of a GOS supplement, to the harvested, pre-conditioned *L. reuteri* strain.

Typically, an "effective amount" of a pre-conditioned probiotic *L. reuteri* strain as defined herein for the composition may comprise a pre-conditioned probiotic *L. reuteri* strain typically in an amount of between 10³ cfu to 10¹² cfu, typically in an amount of between 10⁴ cfu to 10¹¹ cfu, preferably in an amount of between 10⁵ cfu to 10¹⁰ cfu or 10⁵ cfu to 10⁹ cfu, likewise preferably in an amount of between 10⁶ cfu to 10⁹ cfu, 10⁶ cfu to 10⁸ cfu or in an amount of 10⁸ cfu to 10¹⁰ cfu. The above-mentioned amounts of pre-conditioned probiotic *L. reuteri* strain are preferably amount of pre-conditioned probiotic *L. reuteri* strain per daily dose. For instance, a dosage form or unit of the probiotic composition preferably comprises the above-mentioned amount of pre-conditioned probiotic *L. reuteri* strain.

A preferred daily dose is around 10⁸ total cfu per daily dose, e.g., 10⁷ to 10⁹ or 10⁸ to 10⁹ cfu per daily dose.

Moreover, an "effective amount" of GOS as defined herein for the composition may comprise GOS in an amount of at least 1 or 2 g, preferably at least 3 g, likewise preferably between 2 to 12 g, or between 2 to 10 g, between 2 to 8 g, or between 2 to 7 g, such as between 3 to 12 g, likewise more preferably between 3 and 10 g, such as between 3 to 8 g, between 3 to 7 g, between 3 to 6 g, or about 2, 3, 4, 5, 6, 7, 8, 9, 10, 11, or 12 g or any range formed thereby. The above-mentioned amounts of GOS are preferably amount of GOS per daily dose. For instance, a dosage form or unit of the probiotic composition preferably comprises the above-mentioned amount of GOS.

The probiotic composition can comprise the pre-conditioned probiotic L. *reuteri* strain in dried form as previously mentioned herein. In such a case, the pre-conditioned *probiotic L. reuteri* strain is preferably in spray-dried or freeze-dried form. Hence, in an embodiment, the probiotic composition comprises an effective amount of a freeze dried, pre-conditioned probiotic L. *reuteri* strain and the effective amount of GOS.

More preferably the probiotic *L. reuteri* strain is *L. reuteri* DSM 17938.

Suitably, the *L. reuteri* strain has at least 99% (suitably, at least 99.1%, at least 99.2%, at least 99.3%, at least 99.4%, at least 99.5%, at least 99.6%, at least 99.7%, at least 99.8%, at least 99.9%) ANI to *L. reuteri* DSM 17938. Preferably, the *L. reuteri* strain has at least 99.9% ANI to *L. reuteri* DSM 17938.

### Examples

The following examples illustrate some specific embodiments of the composition for use according to the present invention. The examples are given solely for the purpose of illustration and are not to be construed as limitations of the present invention, as many variations thereof are possible without departing from the spirit of the invention.

### Example 1 - Method for pre-conditioning L. reuteri with GOS

According to example 1 of WO2022/191767 A1, *L. reuteri frozen* starter cultures *(L. reuteri* DSM 17938) were used to inoculate a fermentation medium containing 4% Bovine milk derived oligosaccharides (BMOS) as carbon source and 4% fructose as electron acceptor (on dry matter). Overnight culture of *L. reuteri* was used to inoculate a fresh fermentation medium containing 6% BMOS as carbon source and 6% fructose as electron acceptor (on dry matter). Fermentation was run under non pH control and at 37°C. Pre-conditioned *L. reuteri* cells were harvested by centrifugation after around 10 h fermentation prior to mixing with protectants and to spray-drying.

BMOS carbohydrate mixture used as source of GOS was composed of 48% GOS, 30% lactose, 8% glucose, 3.9% galactose, and >0.2% 3'-SL + 6'-SL (g/100 g of dry matter).

*L. reuteri* cell counts were determined by pour plating. Briefly, serial decimal dilutions spray dried samples were performed in tryptone salt (Oxoid, LP0042) solution. Subsequently, 100 L of the appropriate dilutions were transferred to Petri dishes and mixed with MRS agar (AES Chemunex, Bruz, France). Analysis was performed in duplicate. Plates were then incubated in aerobic conditions at 37°C for 48 h. Colony forming units per gram (cfu/g) were calculated from the number of colonies counted on plates with appropriate dilution by determining their arithmetic mean.

Furthermore, according to an optional step, highly purified GOS (>93%) was added to the harvested pre-conditioned probiotic *L. reuteri.*

### Example 2 - Infant formula

An example of the composition of an infant formula according to the present invention is given in the below table 1. This composition is given by way of illustration only.

An example of the composition of an infant formula not according to the present invention is given in the below table 2.

In tables 1 and 2, /100g means each ingredient /100g of the powdered infant formula.

The relevant values of the draft of the Codex Standard For Follow-Up Formula For Older Infants And Product For Young Children CXS 156-1987 aged 6-36 months, report dated March 2023, appendix 2 from page 30, which is publicly available, is given in the below table 3.

**Table 1. Composition of the infant formula YCF**

| | **Unit** | **/100g** | **/100kcal** |
|---|---|---|---|
| Energy | kcal | 478 | 100 |
| Protein | g | 10.6 | 2.21 |
| Carbohydrates, incl. lactose | g | 59 | 12.36 |
| Fat | g | 22.0 | 4.60 |
| Vitamin A | IU | 2310 | 483.26 |
| Vitamin B1 | mg | 0.68 | 0.14 |
| Vitamin B2 | mg | 1.35 | 0.28 |
| Niacin B3 | mg | 6.08 | 1.27 |
| Vitamin B6 | mg | 0.53 | 0.11 |
| Vitamin B12 | µg | 1.61 | 0.34 |
| Vitamin C | mg | 134 | 28.03 |
| Vitamin D | IU | 420 | 87.87 |
| Vitamin E | IU | 15.30 | 3.20 |
| Vitamin K | µg | 41.00 | 8.58 |
| Biotin B7 | µg | 19.44 | 4.07 |
| Pantothenic Acid B5 | mg | 3.75 | 0.78 |
| Folic Acid B9 | µg | 126.00 | 26.36 |
| Taurine | mg | 1.45 | 0.30 |
| Calcium | mg | 865.00 | 180.96 |
| Phosphorous | mg | 470.00 | 98.33 |
| Magnesium | mg | 70.00 | 14.64 |
| Iron | mg | 7.00 | 1.46 |
| Zinc | mg | 4.40 | 0.92 |
| Selenium | µg | 15.00 | 3.14 |
| Potassium | mg | 750.00 | 156.90 |
| Sodium | mg | 260.00 | 54.39 |
| Prebiotic oligosaccharides incl. GOS | g | 2.90 | 0.61 |
| Probiotic of example 1 | cfu/g | 1x10⁷ | 2x10⁶ |
| Iodine | µg | 112.00 | 23.43 |
| Manganese | µg | 150.00 | 31.38 |
| Chloride | mg | 364.00 | 76.15 |
| Copper | µg | 0.40 | 0.08 |
| Choline | mg | 63.00 | 13.125 |

| | | | |
|---|---|---|---|
| 1 IU for vitamin D is the biological equivalent of 0.025 microgram of cholecalciferol or ergocalciferol. 1 IU for vitamin A is the biological equivalent 0.3 microgram of retinol or of beta-carotene. 1 IU for vitamin E is the biological equivalent of 0.67 mg of d-alpha-tocopherol, or of 0.9 mg of dl-alpha-tocopherol. | | | |

**Table 2. Composition of the infant formula CTRL**

| | **Unit** | **/100g** | **/100kcal** |
|---|---|---|---|
| Energy | kcal | 478 | 100 |
| Protein | g | 15.8 | 3.29 |
| Carbohydrates. incl. lactose | g | 56 | 11.65 |
| Fat | g | 21.0 | 4.39 |
| Vitamin A | IU | 2040.0 | 426.78 |
| Vitamin B1 | mg | 1.00 | 0.21 |
| Vitamin B2 | mg | 1.1 | 0.23 |
| Niacin B3 | mg | 1.3 | 0.27 |
| Vitamin B6 | mg | 1.5 | 0.31 |
| Vitamin B12 | µg | 3.2 | 0.68 |
| Vitamin C | mg | 80.0 | 16.74 |
| Vitamin D | IU | 122.0 | 25.52 |
| Vitamin E | IU | 11.0 | 2.30 |
| Vitamin K | µg | 22.0 | 4.60 |
| Biotin B7 | µg | 9.4 | 1.97 |
| Pantothenic Acid B5 | mg | 2.0 | 0.42 |
| Folic Acid B9 | µg | 50.0 | 10.46 |
| Taurine | mg | - | 0.00 |
| Calcium | mg | 742 | 155.23 |
| Phosphorous | mg | 542.00 | 113.39 |
| Magnesium | mg | 58.00 | 12.13 |
| Iron | mg | 8.52 | 1.78 |
| Zinc | mg | 5.72 | 1.20 |
| Selenium | µg | 6.76 | 1.41 |
| Potassium | mg | 860.00 | 179.92 |
| Sodium | mg | 310.00 | 64.85 |
| Prebiotics Oligosaccharides | g | - | - |
| Probiotics | cfu/g | - | - |
| Iodine | µg | 112.00 | 23.43 |
| Manganese | µg | 66.00 | 13.81 |
| Chloride | mg | 480.00 | 100.42 |
| Copper | µg | 0.51 | 0.11 |
| Choline | mg | 79.00 | 16.53 |

The IU are as defined in Table 1.

**Table 3. Draft of the Codex Standard For Follow-Up Formula For Older Infants And Product For Young Children CXS 156-1987 aged 6-36 months, report dated March 2023, appendix 2 from page 30, which is publicly available**

| | | **Max. /100kcal** | **Max. /100kcal** |
|---|---|---|---|
| Energy | kcal | | |
| Protein | g | 1.80 | |
| Carbohydrates | g | | 12.5 |
| Fat | g | 3.50 | |
| Vitamin A | IU | 250.00 | 600.00 |
| Vitamin B1 | mg | 0.04 | |
| Vitamin B2 | mg | 0.08 | |
| Niacin B3 | mg | 0.300 | 1.500 |
| Vitamin B6 | mg | 0.05 | |
| Vitamin B12 | µg | 0.15 | |
| Vitamin C | mg | 10.00 | 70.00 |
| Vitamin D | IU | 60.00 | 120.00 |
| Vitamin E | IU | 1.34 | 13.4 |
| Vitamin K | µg | 4.00 | 27.00 |
| Biotin B7 | µg | 1.50 | 10 |
| Pantothenic Acid B5 | mg | 0.40 | |
| Folic Acid B9 | µg | 10.00 | |
| Taurine | mg | | |
| Calcium | mg | 90.00 | 280.00 |
| Phosphorous | mg | 60.00 | 575.44 |
| Magnesium | mg | 5.00 | 15.00 |
| Iron | mg | 1.00 | 2.00 |
| Zinc | mg | 0.50 | 1.09 |
| Selenium | µg | 2.00 | 9.00 |
| Potassium | mg | 80.00 | 436.91 |
| Sodium | mg | 20.00 | 85.00 |
| Prebiotics oligosaccharides | g | | |
| Probiotics | cfu/g | 6x10⁵ | |
| Iodine | µg | 5.00 | 29.84 |
| Manganese | µg | 1.00 | 100 |
| Chloride | mg | 55.00 | 282.39 |
| Copper | µg | | |
| Choline* | mg | | 240.00 |

The IU are as defined in Table 1.

### Example 3 - Clinical study

### Study population

Healthy toddlers between 24 months (±1 week) to 36 months (±1 week) of age not consuming pre- or probiotics in the past month and without history of muscle disorder, malabsorption, metabolic, congenital, or chromosomal abnormality.

### Randomized, double-blind controlled clinical trial in one study center in the Philippines

The new young child formula (YCF) was clinically tested in toddlers and the study's objectives were the outcomes in terms of muscle strength evaluated by handgrip, vitamin status (serum vitamin D, B3, B6, B9), microbiota diversity and bacteria composition.

Children were randomized to receive one of two study milks and advised to consume 2 servings/day (~235 mL/serving) of the study milks for 6 months:
Control Milk (CTRL) which was a minimally fortified powdered milk mimicking local traditional milk, or
Experimental Milk (EXPL) which was a powdered cow's milk based Young Child Formula (YCF) fortified with micronutrients and containing galacto-oligosaccharides (GOS) (4 g/L) supplemented with preconditioned *L. reuteri* DSM 17938 (at concentration that guarantees 108 cfu/day), a fat blend (oil mixture) comprising fatty acid (FA) triacylglycerols (TAG), said fatty acid TAG esters having low levels of stearic, palmitic and myristic esters at the sn-1(3) position, and specific levels of short chain fatty acids (SCFA ) and monounsaturated fatty acids (MUFA), vitamin B3, vitamin D and vitamin B9, and minerals magnesium, manganese and selenium.

An unblinded reference group (REF) of habitual intake was also enrolled to provide a "real-world" comparison.

Ingredients of interest for EXPL and CTRL are listed in **Table 4.**

The specific composition of the oil mixtures is listed in **Table 5.**

**Table 4. Vitamin B3, vitamin D and vitamin B9, magnesium, manganese and selenium, GOS, preconditioned L. reuteri DSM 17938, and oil mix content in Experimental Milk (EXPL) and Young Child Formula (YCF).**

| | EXPL | | CTRL | |
|---|---|---|---|---|
| | **/100mL** | **/day** | **/100mL** | **/day** |
| Vitamin D (IU) | 60 | 280 | 18.2 | 85 |
| Vitamin B3 (mg) | 6.1 | 30.5 | 1.3 | 6.4 |
| Vitamin B9 (µg) | 126 | 630 | 50 | 250 |
| Magnesium (mg) | 9.5 | 44.4 | 8.1 | 37.8 |
| Manganese (µg) | 23.7 | 110 | 9.1 | 43 |
| Selenium (µg) | 2.09 | 9.7 | 0.94 | 4.4 |
| GOS (g) | 0.4 | 2 | - | - |
| *preconditioned* L. reuteri (cfu) | 1.4x10⁶ | Approx. 10⁸ | - | - |
| Oil mixture | OM(EXPL) | | OM(CTRL) | |

The IU is as defined in Table 1.

**Table 5. Compositions of the oil mixtures**

| **Oil (wt.-%)** | **OM(CTRL)** | **OM(EXPL)** |
|---|---|---|
| Coconut oil | 21,0 | 17,0 |
| Corn oil | - | - |
| Palm kernel oil | - | - |
| Palm olein | 46,6 | - |
| Rapeseed oil | 21,1 | 16,4 |
| Sunflower oil | 11,3 | 13,0 |
| high oleic sunflower oil | - | 53,6 |
| Sum C14:0+C16:0+C18:0 (g/100 g Oil Mix) | 27,2 | 11,0 |
| *sn*-1,3 C14:0+C16:0+C18:0 (%) | 90,3 | 93,5 |
| *sn*-1,3 C14:0+C16:0+C18:0 (g/100 g Oil Mix) | 24,6 | 10,3 |
| C14:0 Myristic Acid | 3,9 | 2,8 |
| C16:0 Palmitic Acid | 20,5 | 4,8 |
| C14:0 Stearic Acid | 3,3 | 3,4 |
| C18:1 n-9 Oleic Acid | 35,5 | 54,2 |
| Sum SCFA | 40,3 | 21,7 |
| Sum MUFA | 35,1 | 54,5 |

### Example 4: Results

### Subject disposition

Of the 273 toddlers who were enrolled in the study, 182 toddlers were randomized to either CTRL or EXPL (n=91 per group) and 91 toddlers continued their habitual diet as the REF group. The retention rate was 77% in both CTRL and EXPL groups in the full analysis set (FAS).

### Baseline characteristics in the Intention-to-treat (ITT) analysis set

Among groups, toddlers were of similar age (mean ± SD: 29.7 ± 3.8 months in CTRL; 29.4 ± 3.4 months in EXPL; and 29. 7± 3.7 months in REF) with similar proportions of males/females at enrollment (53% females in all three groups).

Most of the toddlers had consumed fortified cow milk in the past day as this was standard feeding practice (87.9% in CTRL, 89.0% in EXPL, and 100% in REF).

### Muscle strength (Handgrip)

Handgrip test (Jamar hand dynamometer) tailored for toddlers have been used as previously described by Bohannon et al. hand grip test ("Handgrip Strength: A Population-Based Study of Norms and Age Trajectories for 3- to 17-Year-Olds." Pediatric Physical Therapy 2 (29): 118-23.) at V1 (baseline) and V3. Toddlers should be seated, shoulders adducted and neutrally rotated, elbow flexed at 90°, forearm in neutral and wrist between 0 and 30° of dorsiflexion. Mean of three trials of grip strength of each hand have been recorded. Repeated measures are to be taken at 1-minute intervals.

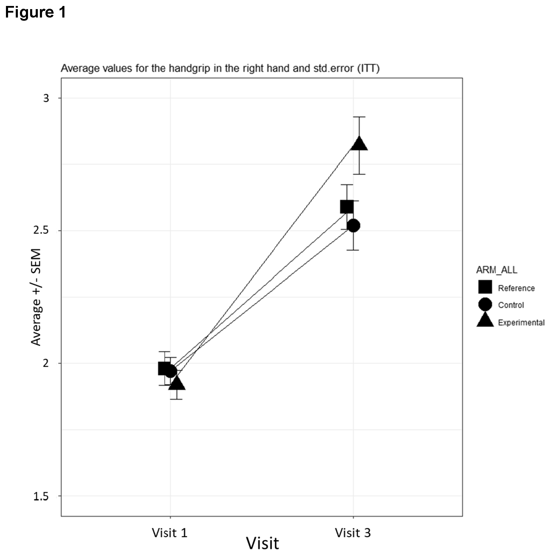

The results are shown in **Figure 1****.**

The handgrip indicates that the subjects treated with EXPL blend according to the invention have increased muscle force (+48.7%) significantly more than the CTRL (+25.8%) and REF (+27.7%) groups (p<0.01).

### Microbiota diversity and composition "bacteria abundance" (Metagenomics)

Gut microbiome analysis for microbiome composition and diversity was performed using next generation shotgun sequencing., a technique that allows to sequence all the genetic material of the gut microbial community and assess inter- and intra-individual differences. Beta diversity analysis using Principal coordinates analysis (PCoA), based on weighted UniFrac distance among three groups indicate that the EXPL group significantly differs from both CTRL and REF groups in terms of microbiome composition.

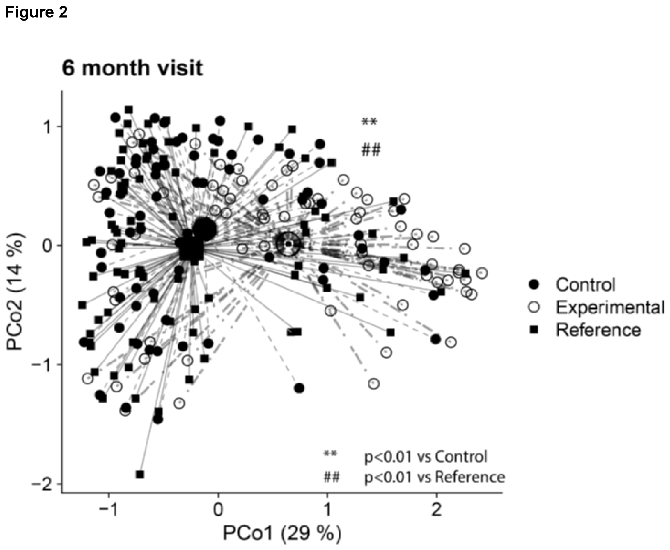

The results are shown in **Figure 2****.**

The summary of the taxonomical microbiome features highlights abundance improvement of *lactobacillus* with enrichment for *L. reuteri* in EXPL compared to CTRL. Both commensals remain unaffected in Control, similar to the reference. *L. reuteri* is almost non-existent in CTRL and REF, with a prevalence below 5%. This result confirms the capacity of the probiotic to persist in the gastrointestinal tract. We cannot distinguish between autochthonous and probiotic for *L. reuteri,* as the methodology does not allow such level of discrimination between strains. But the expansion of *L. reuteri* only in the EXPL cohort suggests that such increase in abundance and prevalence it is promoted by the formula.

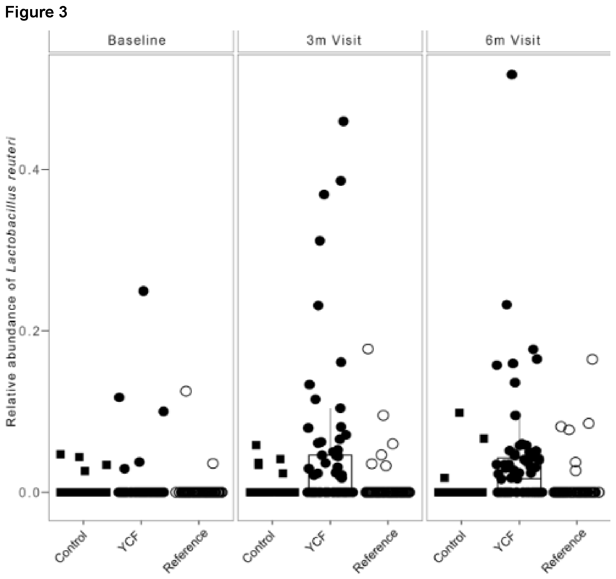

The results are shown in **Figure 3****.**

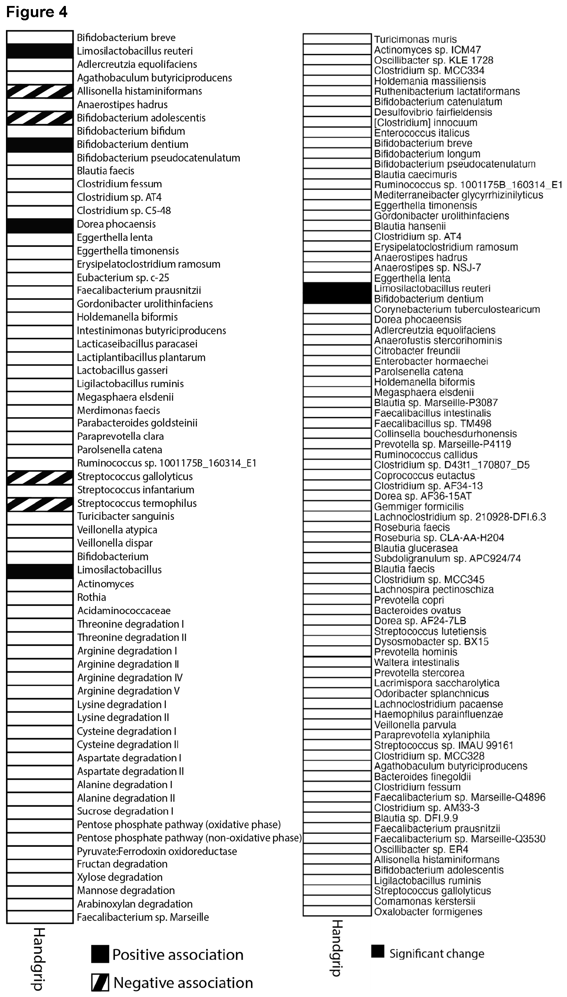

Non-parametric Kendall correlation analysis from all infants at all visits (n=676) investigating the association between clinical outcomes and abundance of microbiome features revealed *L. reuteri, B. dentium, dorea phocaeensis* and *Limosilactobacillus* at genus level to be significantly positively associated with muscle handgrip and hence muscle force. The heatmap shown in **Figure 4** is summarizing correlations between clinical variables and microbiome taxa at species level in EXPL group.

Cross feeding opportunity through fiber or intermediate metabolite can explain the cocktail of bacteria associated with muscle force. As an example, *B. dentium* abundancy increase as been described to be obtain by cross feeding of fiber by *L. reuteri* through acetate and other intermediate metabolite.

### Vitamin D levels (serum vitamin D levels & prevalence of vitamin D insufficiency)

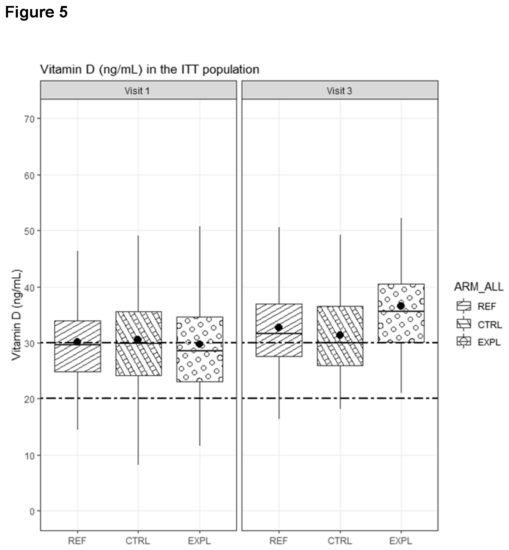

Vitamin D in serum was measured as 25 hydroxy vitamin D at Visit 1 (Baseline) and Visit 3 (6 months). After 6 months of intervention, Vitamin D levels were significantly higher in EXPL in comparison to both the CTRL and REF groups **(****Figure 5****).** Mean serum Vitamin D values (log-transformed) were 17% (95% Cl: 11%, 23%; P<0.0001) higher than the vitamin D measurements in CTRL, and 10% (95% Cl: 5%, 15%; P<0.001) higher than the vitamin D measurements in REF.

The Pediatric Endocrine Society proposed < 30 ng/mL as insufficiency and the American Academy of Pediatrics classified 25 hydroxy vitamin D concentration < 20 ng/mL as deficiency (Chang 2019). At enrolment, roughly 50% of the subjects were vitamin D insufficient in all three arms. After 6 months of feeding, only 25% of the subjects in the EXPL group were below the threshold of insufficiency, compared to 49% of the subjects in the CTRL group and 43% in the REF group. Similarly, at enrolment 12% of the subjects were vitamin D deficient in the EXPL group, 3% in CTRL and 4% in REF. After 5 months of feeding, no subject from the EXPL group was classified as vitamin D deficient but still 4% in CTRL and 3% in REF groups

### (Figure 6).

The vitamin D status in children consuming YCF containing preconditioned *L. reuteri* was significantly improved after 6 months of feeding.

### Vitamin B9 (plasma levels and association with handgrip)

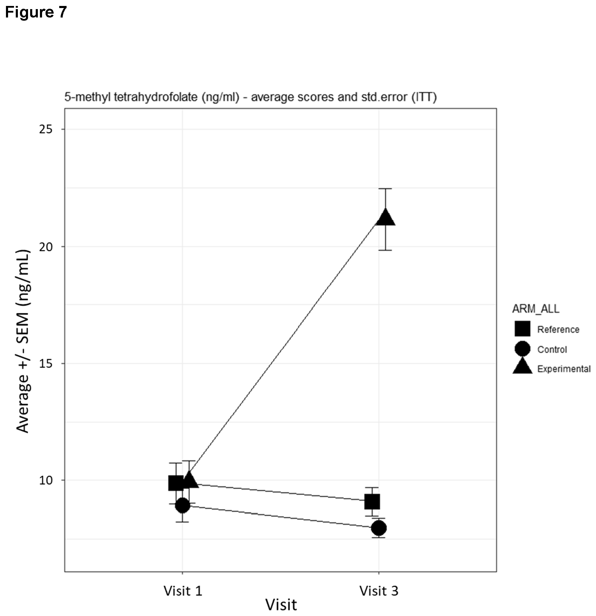

Vitamin B9 in serum was measured as 5-methyl tetrahydrofolate (active form) at Visit 1 (Baseline) and Visit 3 (6 months). After 6 months of intervention, Vitamin B9 levels were significantly higher in EXPL in comparison to both the CTRL and REF groups **(****Figure 7****).** Mean serum Vitamin B9 (5-methyl tetrahydrofolate) values (log-transformed) were 86% (95% Cl: 69%, 103%; P<0.0001) higher than the vitamin B9 (5-methyl tetrahydrofolate) measurements in CTRL, and 80% (95% Cl: 67%, 96%; P<0.001, Model 1) higher than the vitamin B9 (5-methyl tetrahydrofolate) measurements in REF.

Various preferred features and embodiments of the present invention will now be described with reference to the following numbered clauses:

### Clauses

1. A nutritional composition comprising *L. reuteri* as a probiotic strain, wherein *L. reuteri* is pre-conditioned.
2. The nutritional composition according to clause 1, wherein *L. reuteri* is pre-conditioned with galacto-oligosaccharide (GOS).
3. The nutritional composition according to clause 1 or clause 2, wherein the pre-conditioned *L. reuteri* is obtained or obtainable by a process comprising the following steps:
   a) cultivating a probiotic *L. reuteri* strain in the presence of galacto-oligosaccharide (GOS) in a growth medium, thereby pre-conditioning the probiotic *L. reuteri* strain;
   b) harvesting the pre-conditioned *L. reuteri* from the growth medium.
4. The nutritional composition according to clause 3, wherein the process is comprising the following further step:
   c) adding (fresh) GOS to the pre-conditioned probiotic *L. reuteri* strain obtained from step b).
5. The nutritional composition according to any of the preceding clauses, further comprising at least one vitamin.
6. The nutritional composition according to clause 5, wherein the at least one vitamin is selected form the list consisting of: vitamin A, vitamin B1, vitamin B2, vitamin B3, vitamin B6, vitamin B12, vitamin C, vitamin D, vitamin E, vitamin K, vitamin B7, vitamin B5, vitamin B9, and any combinations thereof; preferably a combination comprising or consisting of high amounts of vitamin B3, vitamin C, vitamin D, vitamin E, vitamin K, vitamin B7, vitamin B5, vitamin B9, wherein the high amount are defined as being at least 30% more than in the draft of the Codex Standard For Follow-Up Formula For Older Infants And Product For Young Children CXS 156-1987 aged 6-36 months, report dated March 2023, appendix 2 from page 30.
7. The nutritional composition according to clause 5 or clause 6, wherein the at least one vitamin is present in the following amount:
   vitamin A, in an amount ranging of from 1000 IU / 100 g to 3000 IU / 100 g, preferably of from 1500 IU / 100 g to 2600 IU / 100 g, more preferably of from 2000 IU / 100 g to 2500 IU / 100 g, wherein one IU for vitamin A is the biological equivalent of 0.3 microgram of retinol or of beta-carotene; and/or
   vitamin B1, in an amount ranging of from 0.1 mg / 100 g to 1 mg / 100 g, preferably of from 0.3 mg / 100 g to 1 mg / 100 g, more preferably of from 0.5 mg / 100 g to 0.9 mg / 100 g; and/or
   vitamin B2, in an amount ranging of from 1 mg / 100 g to 3 mg / 100 g, preferably of from 1 mg / 100 g to 2 mg / 100 g, more preferably of from 1.1 mg / 100 g to 1.6 mg / 100 g; and/or vitamin B3, in an amount ranging of from 1.5 mg / 100 g to 12 mg / 100 g, preferably of from 3 mg / 100 g to 10 mg / 100 g, more preferably of from 4 mg / 100 g to 8 mg / 100 g; and/or
   vitamin B6, in an amount ranging of from 0 mg / 100 g to 1.3 mg / 100 g, preferably of from 0.2 mg / 100 g to 1 mg / 100 g, more preferably of from 0.3 mg / 100 g to 0.7 mg / 100 g; and/or
   vitamin B12, in an amount ranging of from 0 microgram / 100 g to 3 microgram / 100 g, preferably of from 1 microgram / 100 g to 2.5 microgram / 100 g, more preferably of from 1 microgram / 100 g to 2 microgram / 100 g; and/or
   vitamin C, in an amount ranging of from 80 mg / 100 g to 400 mg / 100 g, preferably of from 85 mg / 100 g to 300 mg / 100 g, more preferably of from 100 mg / 100 g to 200 mg / 100 g; and/or
   vitamin D, in an amount ranging of from 130 IU / 100 g to 900 IU / 100 g, preferably of from 200 IU / 100 g to 700 IU / 100 g, more preferably of from 350 IU / 100 g to 500 IU / 100 g, wherein one IU for vitamin D is the biological equivalent of 0.025 microgram of cholecalciferol or ergocalciferol; and/or
   vitamin E, in an amount ranging of from 11 IU / 100 g to 30 IU / 100 g, preferably of from 12 IU / 100 g to 25 IU / 100 g, more preferably of from 12 IU / 100 g to 20 IU / 100 g, wherein one IU for vitamin E is the biological equivalent of 0.67 mg of d-alpha-tocopherol, or of 0.9 mg of dl-alpha-tocopherol; and/or
   vitamin K, in an amount ranging of from 23 microgram / 100 g to 100 microgram / 100 g, preferably of from 25 microgram / 100 g to 80 microgram / 100 g, more preferably of from 25 microgram / 100 g to 70 microgram / 100 g; and/or
   vitamin B7, in an amount ranging of from 9 microgram / 100 g to 100 microgram / 100 g, preferably of from 12 microgram / 100 g to 60 microgram 100 g, more preferably of from 15 microgram / 100 g to 30 microgram / 100 g; and/or
   vitamin B5, in an amount ranging of from 2.1 mg / 100 g to 10 mg / 100 g, preferably of from 2.5 mg / 100 g to 8 mg / 100 g, more preferably of from 3 mg / 100 g to 6 mg / 100 g; and/or
   vitamin B9, in an amount ranging of from 51 microgram / 100 g to 350 microgram / 100 g, preferably of from 70 microgram / 100 g to 300 microgram / 100 g, more preferably of from 90 microgram / 100 g to 200 microgram / 100 g.
8. The nutritional composition according to any of clauses 5 to 7, wherein the at least one vitamin is present in the following amount:
   vitamin A, in an amount ranging of from 200 IU / 100 kcal to 1000 IU / 100 kcal, preferably of from 300 IU / 100 kcal to 800 IU / 100 kcal, more preferably of from 300 IU / 100 kcal to 600 IU / 100 kcal, wherein one IU for vitamin A is the biological equivalent of 0.3 microgram of retinol or of beta-carotene; and/or
   vitamin B1, in an amount ranging of from 0.02 mg / 100 kcal to 0.5 mg / 100 kcal, preferably of from 0.05 mg / 100 kcal to 0.4 mg / 100 kcal, more preferably of from 0.05 mg / 100 kcal to 0.2 mg / 100 kcal; and/or
   vitamin B2, in an amount ranging of from 0.02 mg / 100 kcal to 0.6 mg / 100 kcal, preferably of from 0.05 mg / 100 kcal to 0.5 mg / 100 kcal, more preferably of from 0.08 mg / 100 kcal to 0.5 mg / 100 kcal; and/or
   vitamin B3, in an amount ranging of from 0.3 mg / 100 kcal to 3 mg / 100 kcal, preferably of from 0.6 mg / 100 kcal to 2.5 mg / 100 kcal, more preferably of from 0.8 mg / 100 kcal to 2 mg / 100 kcal; and/or
   vitamin B6, in an amount ranging of from 0 mg / 100 kcal to 0.3 mg / 100 kcal, preferably of from 0.05 mg / 100 kcal to 0.25 mg / 100 kcal, more preferably of from 0.05 mg / 100 kcal to 0.20 mg / 100 kcal; and/or
   vitamin B12, in an amount ranging of from 0 microgram / 100 kcal to 0.65 microgram / 100 kcal, preferably of from 0.05 microgram / 100 kcal to 0.6 microgram / 100 kcal, more preferably of from 0.05 microgram / 100 kcal to 0.5 microgram / 100 kcal; and/or
   vitamin C, in an amount ranging of from 17 mg / 100 kcal to 100 mg / 100 kcal, preferably of from 20 mg / 100 kcal to 80 mg / 100 kcal, more preferably of from 20 mg / 100 kcal to 50 mg / 100 kcal; and/or
   vitamin D, in an amount ranging of from 26 IU / 100 kcal to 150 IU / 100 kcal, preferably of from 40 IU / 100 kcal to 120 IU / 100 kcal, more preferably of from 50 IU / 100 kcal to 130 IU / 100 kcal, wherein one IU for vitamin D is the biological equivalent of 0.025 microgram of cholecalciferol or ergocalciferol; and/or
   vitamin E, in an amount ranging of from 2.0 IU / 100 kcal to 12 IU / 100 kcal, preferably of from 2.5 IU / 100 kcal to 10 IU / 100 kcal, more preferably of from 2.6 IU / 100 kcal to 5 IU / 100 kcal, wherein one IU for vitamin E is the biological equivalent of 0.67 mg of d-alpha-tocopherol, or of 0.9 mg of dl-alpha-tocopherol; and/or
   vitamin K, in an amount ranging of from 4.6 microgram / 100 kcal to 25 microgram / 100 kcal, preferably of from 5 microgram / 100 kcal to 20 microgram / 100 kcal, more preferably of from 5 microgram / 100 kcal to 15 microgram / 100 kcal; and/or
   vitamin B7, in an amount ranging of from 2 microgram / 100 kcal to 15 microgram / 100 kcal, preferably of from 2.5 microgram / 100 kcal to 12 microgram / 100 kcal, more preferably of from 3 microgram / 100 kcal to 10 microgram / 100 kcal; and/or
   vitamin B5, in an amount ranging of from 0.43 mg / 100 kcal to 2 mg / 100 kcal, preferably of from 0.5 mg / 100 kcal to 1.6 mg / 100 kcal, more preferably of from 0.55 mg / 100 kcal to 1.2 mg / 100 kcal; and/or
   vitamin B9, in an amount ranging of from 13 microgram / 100 kcal to 80 microgram / 100 kcal, preferably of from 15 microgram / 100 kcal to 60 microgram / 100 kcal, more preferably of from 20 microgram / 100 kcal to 45 microgram / 100 kcal.
9. The nutritional composition according to any of clauses 6 to 8, comprising high amounts of the following at least one vitamin, wherein the high amounts are preferably defined as in anyone of clause 6 or clause 7:
   - vitamin B3;
   - vitamin D;
   - vitamin B9;
   - vitamin B3, vitamin D and vitamin B9;
   - vitamin B3 and vitamin D;
   - vitamin B3 and vitamin B9;
   - vitamin D and vitamin B9;
   - vitamin B3 and vitamin B7;
   - vitamin D and vitamin B7;
   - vitamin B9 and vitamin B7;
   - vitamin B3, vitamin D and vitamin B7;
   - vitamin B3, vitamin D, vitamin B9 and vitamin B7;
   - vitamin B3, vitamin D and vitamin K;
   - vitamin B3, vitamin D, vitamin B7 and vitamin K;
   - vitamin B3, vitamin D, vitamin B7, vitamin B9 and vitamin K;
   - vitamin B3, vitamin D, vitamin B7, vitamin B9, vitamin C and vitamin K;
   - vitamin B3, vitamin D, vitamin B7, vitamin B9, vitamin C, vitamin E and vitamin K;
   - vitamin D and vitamin B5;
   - vitamin B9 and vitamin B5;
   - vitamin B3, vitamin D, vitamin B5 and vitamin B9;
   - vitamin B3, vitamin D and vitamin B5;
   - vitamin B3, vitamin B9 and vitamin B5;
   - vitamin D, vitamin B9 and vitamin B5;
   - vitamin B3, vitamin D, vitamin B7, vitamin B9, vitamin C, vitamin **E,** vitamin K and vitamin B5;
      preferably comprising high amounts of the following at least one vitamin, wherein the high amounts are preferably defined as in anyone of clause 6 or clause 7:
   - vitamin D;
   - vitamin B3, vitamin D and vitamin B9;
   - vitamin B3 and vitamin D;
   - vitamin D and vitamin B9;
   - vitamin D and vitamin B7;
   - vitamin B3, vitamin D and vitamin B7;
   - vitamin B3, vitamin D, vitamin B9 and vitamin B7;
   - vitamin B3, vitamin D and vitamin K;
   - vitamin B3, vitamin D, vitamin B7 and vitamin K.
10. The nutritional composition according to any of the preceding clauses, further comprising lipids comprising fatty acid (FA) triacylglycerols (TAG).
11. The nutritional composition according to clause 10, said lipids comprising fatty acid (FA) triacylglycerols (TAG), said fatty acid TAG comprising stearic, palmitic and myristic esters, wherein the sum of the amount of stearic, palmitic and myristic esters at the sn-1(3) position of the TAG is less than 13.0 wt-% of TAG and the myristic acid represents up to 3.7 wt-% of the total FA, the palmitic acid represents up to 11.1 wt-% of the total FA and stearic acid represents up to 4.6 wt-% of the total FA, wherein the short chain fatty acid (SCFA) are present in preferably less than 40 wt-%, wherein the mono-unsaturated fatty acids (MUFA) are present in preferably more than 35.1 wt-%.
12. The nutritional composition according to clause 10 or clause 11, wherein the lipids comprise a mixture of oils selected from:
   16.9 wt-% to 22.0 wt-% of rapeseed oil, 11.5 to 14.5 wt-% of sunflower oil, 40.0 to 58 wt-% of high oleic sunflower oil, and 15.0 wt-% to 20.5 wt-% of coconut oil.
13. The nutritional composition according to any of the preceding clauses, further comprising at least one mineral.
14. The nutritional composition according to clause 13, wherein the at least one mineral is selected form the list consisting of: calcium, phosphorous, magnesium, iron, zinc, potassium, copper, manganese, selenium, sodium and any combinations thereof; preferably selenium, manganese, a combination comprising or consisting of selenium, manganese, or a combination comprising or consisting of magnesium, manganese, selenium.
15. The nutritional composition according to clause 13 or clause 14, wherein the at least one mineral is present in the following amount:
   magnesium, in an amount ranging of from 30 mg / 100 g to 200 mg / 100 g, preferably of from 40 mg / 100 g to 150 mg / 100 g, more preferably of from 15 mg / 100 g to 150 mg / 100 g;
      and/or
   manganese, in an amount ranging of from 70 microgram / 100 g to 800 microgram / 100 g, preferably of from 100 microgram / 100 g to 450 microgram / 100 g, more preferably of from 100 microgram / 100 g to 250 microgram / 100 g; and/or
   selenium, in an amount ranging of from 7 microgram / 100 g to 120 microgram / 100 g, preferably of from 10 microgram / 100 g to 50 microgram / 100 g, more preferably of from 10 microgram / 100 g to 25 microgram / 100 g;
      and/or
   magnesium in an amount ranging of from 6 mg / 100 kcal to 30 mg / 100 kcal, preferably of from 8 mg / 100 kcal to 25 mg / 100 kcal, more preferably of from 10 mg / 100 kcal to 15 mg / 100 kcal; and/or
   manganese in an amount ranging of from 15 microgram / 100 kcal to 250 microgram / 100 kcal, preferably of from 20 microgram / 100 kcal to 200 microgram / 100 kcal, more preferably of from 20 microgram / 100 kcal to 70 microgram / 100 kcal; and/or
   selenium in an amount ranging of from 2.5 microgram / 100 kcal to 12 microgram / 100 kcal, preferably of from 2.8 microgram / 100 kcal to 9 microgram / 100 kcal, more preferably of from 3.0 microgram / 100 kcal to 9 microgram / 100 kcal.
16. The nutritional composition according to any of the preceding clauses, further comprising at least one further probiotic.
17. The nutritional composition according to clause 16, wherein the at least one further probiotic is selected form the list consisting of a *Dorea, a Bifidobacterium,* and any combination thereof.
18. The nutritional composition according to clause 16 or clause 17, wherein the at least one further probiotic is a *Bifidobacterium* selected form the list consisting of: *Bifidobacterium dentium, Bifidobacterium infantis,* and combination thereof.
19. The nutritional composition according to clause 16, wherein the at least one further probiotic is a *Limosilactobacillus.*
20. The nutritional composition according to clause 16 or clause 17, wherein the at least one further probiotic is *Dorea phocaeensis.*
21. The nutritional composition according to any of the preceding clauses, wherein the nutritional composition is an infant formula, a starter infant formula, a follow-on or follow-up infant formula, a baby food, an infant cereal composition, a growing-up milk, a fortifier or a supplement.
22. The nutritional composition according to any of the preceding clauses, for use in improving and/or increasing and/or maintaining and/or treating and/or stimulating and/or promoting and/or preventing at least one muscle condition in a subject.
23. The nutritional composition for use according to clause 22, wherein the subject is an infant, a young child, or a child.
24. The nutritional composition for use according to clause 23, wherein the subject was born by C- section and/or preterm, and/or small for gestational age (SGA) and/or with a low, very low, or extremely low weight at birth (LWB, VLBW, or ELBW) and/or experienced intra-uterine growth retardation (IUGR).
25. The nutritional composition for use according to any of clauses 22 to 24, for use in improving and/or increasing and/or maintaining and/or treating and/or stimulating and/or promoting and/or preventing at least one muscle condition in a subject, selected form the list consisting of:
   increasing muscle strength;
   increasing muscle force;
   increasing grip strength;
   improving endurance capacity;
   increasing muscle quality;
   increasing or maintaining muscle mass;
   increasing muscle specific force;
   increasing exercise capacity;
   modulating muscle health;
   increasing muscle force generation;
   treating a muscle disorder;
   preventing a muscle disorder;
   (promoting muscle health);
   promoting normal muscle function;
   modulating muscle stem cells function;
   promoting muscle stem cell function;
   increasing muscle stem cell function;
   and any combinations thereof;
26. The nutritional composition for use according to any of clauses 22 to 25, for use in increasing muscle strength and/or increasing muscle force and/or increasing grip strength and/or increasing or maintaining muscle mass in a subject.
27. The nutritional composition for use according to any of clauses 22 to 25, for use in treating a muscle disorder in a subject, such as infant, a young child or a child.
28. Use of a nutritional composition according to any of clauses 1 to 21, for improving and/or increasing and/or maintaining and/or treating and/or stimulating and/or promoting at least one muscle condition in a subject.
29. A method of improving and/or increasing and/or maintaining and/or treating and/or stimulating and/or promoting at least one muscle condition in a subject, comprising a step of administrating a nutritional composition according to any of the clauses 1 to 21 to the subject.
30. A process of using a nutritional composition according to any of the clauses 1 to 21 for improving and/or increasing and/or maintaining and/or treating and/or stimulating and/or promoting at least one muscle condition in a subject, comprising a step of administrating the nutritional composition to the subject.
31. The use according to clause 28 or the method according to clause 29 or the process of using according to clause 30, wherein the muscle condition is as defined in any of clauses 25 to 27, and/or wherein the subject is as defined in clause 23 or clause 24.
32. The nutritional composition according to any of clauses 1 to 15, or to clause 21, for use in increasing the abundance of at least one probiotic in the gut of a subject.
33. The nutritional composition according to clause 32, wherein the at least one further probiotic is selected form the list consisting of a Dorea, a Bifidobacterium, and any combination thereof.
34. The nutritional composition according to clause 32 or clause 33, wherein the at least one further probiotic is a Bifidobacterium selected form the list consisting of: Bifidobacterium dentium, Bifidobacterium infantis, and combination thereof.
35. The nutritional composition according to clause 32, wherein the at least one further probiotic is a *Limosilactobacillus.*
36. The nutritional composition according to clause 32 or clause 33, wherein the at least one further probiotic is *Dorea phocaeensis.*
37. The nutritional composition according to any of clauses 1 to 21, for use in treating at least one muscle condition in a subject.
38. The nutritional composition according to any of clauses 1 to 21, for use in treating at least one muscle disorder in a subject, wherein the subject is preferably an infant, a young child or a child.
39. The nutritional composition according to any of clauses 1 to 21, for use in preventing at least one muscle disorder in a subject, such as an infant, a young child or a child.
40. The nutritional composition according to any of clauses 1 to 21, for use in improving and/or increasing at least one muscle condition in a subject, wherein the subject is infant, a young child or a child.
41. The nutritional composition according to any of clauses 1 to 21, for use in stimulating at least one muscle condition in a subject, wherein the subject is infant, a young child or a child.
42. The nutritional composition according to any of clauses 1 to 21, for use in improving and/or increasing at least one muscle condition in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).
43. The nutritional composition according to any of clauses 1 to 21, for use in improving and/or increasing at least one muscle condition in a subject, wherein the subject is an infant, a young child or a child, or older than a child or a teenager, an adult, including elderly, recovering from a disease, notably from a muscle disorder.
44. The nutritional composition according to any of clauses 1 to 21, for use in improving and/or increasing at least one muscle condition in a subject, wherein the subject is an infant, a young child or a child, or older than a child like a teenager, an adult, including elderly, suffering from a disease, notably from a muscle disorder.
45. The nutritional composition according to any of clauses 1 to 21, for use in improving and/or increasing at least one muscle condition in a subject, wherein the subject is elderly, notably with sarcopenia and/or frailty.
46. The nutritional composition according to any of clauses 1 to 21, for use in stimulating at least one muscle condition in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).
47. The nutritional composition according to any of clauses 1 to 21, for use in stimulating at least one muscle condition in a subject, wherein the subject is an infant, a young child or a child, or older than a child or a teenager, an adult, including elderly, recovering from a disease, notably from a muscle disorder.
48. The nutritional composition according to any of clauses 1 to 21, for use in stimulating at least one muscle condition in a subject, wherein the subject is an infant, a young child or a child, or older than a child like a teenager, an adult, including elderly, suffering from a disease, notably from a muscle disorder.
49. The nutritional composition according to any of clauses 1 to 21, for use in stimulating at least one muscle condition in a subject, wherein the subject is elderly, notably with sarcopenia and/or frailty.
50. The nutritional composition according to any of clauses 1 to 21, for use in promoting at least one muscle condition in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).
51. The nutritional composition according to any of clauses 1 to 21, for use in promoting at least one muscle condition in a subject, wherein the subject is an infant, a young child or a child, or older than a child or a teenager, an adult, including elderly, recovering from a disease, notably from a muscle disorder.
52. The nutritional composition according to any of clauses 1 to 21, for use in promoting at least one muscle condition in a subject, wherein the subject is an infant, a young child or a child, or older than a child like a teenager, an adult, including elderly, suffering from a disease, notably from a muscle disorder.
53. The nutritional composition according to any of clauses 1 to 21, for use in promoting at least one muscle condition in a subject, wherein the subject is elderly, notably with sarcopenia and/or frailty.
54. The nutritional composition according to any of clauses 1 to 21, for use in increasing muscle strength in a subject, wherein the subject is infant, a young child or a child.
55. The nutritional composition according to any of clauses 1 to 21, for use in increasing muscle strength in a subject, wherein the subject is infant, a young child or a child born by C-section and/or preterm, and/or small for gestational age (SGA) and/or with a low, very low, or extremely low weight at birth (LWB, VLBW, or ELBW) and/or experienced intra-uterine growth retardation (IUGR).
56. The nutritional composition according to any of clauses 1 to 21, for use in increasing muscle strength in a subject, wherein the subject is an infant, a young child or a child, or older than a child or a teenager, an adult, including elderly, recovering from a disease, notably from a muscle disorder.
57. The nutritional composition according to any of clauses 1 to 21, for use in increasing muscle strength in a subject, wherein the subject is an infant, a young child or a child, or older than a child like a teenager, an adult, including elderly, suffering from a disease, notably from a muscle disorder.
58. The nutritional composition according to any of clauses 1 to 21, for use in increasing muscle strength in a subject, wherein the subject is elderly, notably with sarcopenia and/or frailty.
59. The nutritional composition according to any of clauses 1 to 21, for use in increasing muscle force in a subject, wherein the subject is infant, a young child or a child.
60. The nutritional composition according to any of clauses 1 to 21, for use in increasing muscle force in a subject, wherein the subject is infant, a young child or a child born by C-section and/or preterm, and/or small for gestational age (SGA) and/or with a low, very low, or extremely low weight at birth (LWB, VLBW, or ELBW) and/or experienced intra-uterine growth retardation (IUGR).
61. The nutritional composition according to any of clauses 1 to 21, for use in increasing muscle force in a subject, wherein the subject is an infant, a young child or a child, or older than a child or a teenager, an adult, including elderly, recovering from a disease, notably from a muscle disorder.
62. The nutritional composition according to any of clauses 1 to 21, for use in increasing muscle force in a subject, wherein the subject is an infant, a young child or a child, or older than a child like a teenager, an adult, including elderly, suffering from a disease, notably from a muscle disorder.
63. The nutritional composition according to any of clauses 1 to 21, for use in increasing muscle force in a subject, wherein the subject is elderly, notably with sarcopenia and/or frailty.
64. The nutritional composition according to any of clauses 1 to 21, for use in increasing grip strength in a subject, wherein the subject is infant, a young child or a child.
65. The nutritional composition according to any of clauses 1 to 21, for use in increasing grip strength in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).
66. The nutritional composition according to any of clauses 1 to 21, for use in increasing grip strength in a subject, wherein the subject is an infant, a young child or a child, or older than a child or a teenager, an adult, including elderly, recovering from a disease, notably from a muscle disorder.
67. The nutritional composition according to any of clauses 1 to 21, for use in increasing grip strength in a subject, wherein the subject is an infant, a young child or a child, or older than a child like a teenager, an adult, including elderly, suffering from a disease, notably from a muscle disorder.
68. The nutritional composition according to any of clauses 1 to 21, for use in increasing grip strength in a subject, wherein the subject is elderly, notably with sarcopenia and/or frailty.
69. The nutritional composition according to any of clauses 1 to 21, for use in improving endurance capacity in a subject, wherein the subject is infant, a young child or a child.
70. The nutritional composition according to any of clauses 1 to 21, for use in increasing muscle quality in a subject, wherein the subject is infant, a young child or a child.
71. The nutritional composition according to any of clauses 1 to 21, for use in increasing muscle quality in a subject, wherein the subject is infant, a young child or a child born by C-section and/or preterm, and/or small for gestational age (SGA) and/or with a low, very low, or extremely low weight at birth (LWB, VLBW, or ELBW) and/or experienced intra-uterine growth retardation (IUGR).
72. The nutritional composition according to any of clauses 1 to 21, for use in increasing muscle quality in a subject, wherein the subject is an infant, a young child or a child, or older than a child or a teenager, an adult, including elderly, recovering from a disease, notably from a muscle disorder.
73. The nutritional composition according to any of clauses 1 to 21, for use in increasing muscle quality in a subject, wherein the subject is an infant, a young child or a child, or older than a child like a teenager, an adult, including elderly, suffering from a disease, notably from a muscle disorder.
74. The nutritional composition according to any of clauses 1 to 21, for use in increasing muscle quality in a subject, wherein the subject is elderly, notably with sarcopenia and/or frailty.
75. The nutritional composition according to any of clauses 1 to 21, for use in increasing muscle mass in a subject, wherein the subject is infant, a young child or a child.
76. The nutritional composition according to any of clauses 1 to 21, for use in increasing muscle mass in a subject, wherein the subject is infant, a young child or a child born by C-section and/or preterm, and/or small for gestational age (SGA) and/or with a low, very low, or extremely low weight at birth (LWB, VLBW, or ELBW) and/or experienced intra-uterine growth retardation (IUGR).
77. The nutritional composition according to any of clauses 1 to 21, for use in increasing muscle mass in a subject, wherein the subject is an infant, a young child or a child, or older than a child or a teenager, an adult, including elderly, recovering from a disease, notably from a muscle disorder.
78. The nutritional composition according to any of clauses 1 to 21, for use in increasing muscle mass in a subject, wherein the subject is an infant, a young child or a child, or older than a child like a teenager, an adult, including elderly, suffering from a disease, notably from a muscle disorder.
79. The nutritional composition according to any of clauses 1 to 21, for use in increasing muscle mass in a subject, wherein the subject is elderly, notably with sarcopenia and/or frailty.
80. The nutritional composition according to any of clauses 1 to 21, for use in increasing muscle specific force in a subject, wherein the subject is infant, a young child or a child.
81. The nutritional composition according to any of clauses 1 to 21, for use in increasing muscle specific force in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).
82. The nutritional composition according to any of clauses 1 to 21, for use in increasing muscle specific force in a subject, wherein the subject is an infant, a young child or a child, or older than a child or a teenager, an adult, including elderly, recovering from a disease, notably from a muscle disorder.
83. The nutritional composition according to any of clauses 1 to 21, for use in increasing muscle specific force in a subject, wherein the subject is an infant, a young child or a child, or older than a child like a teenager, an adult, including elderly, suffering from a disease, notably from a muscle disorder.
84. The nutritional composition according to any of clauses 1 to 21, for use in increasing muscle specific force in a subject, wherein the subject is elderly, notably with sarcopenia and/or frailty.
85. The nutritional composition according to any of clauses 1 to 21, for use in modulating muscle health in a subject.
86. The nutritional composition according to any of clauses 1 to 21, for use in increasing muscle force generation in a subject, wherein the subject is infant, a young child or a child.
87. The nutritional composition according to any of clauses 1 to 21, for use in increasing muscle force generation in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).
88. The nutritional composition according to any of clauses 1 to 21, for use in increasing muscle force generation in a subject, wherein the subject is an infant, a young child or a child, or older than a child or a teenager, an adult, including elderly, recovering from a disease, notably from a muscle disorder.
89. The nutritional composition according to any of clauses 1 to 21, for use in increasing muscle force generation in a subject, wherein the subject is an infant, a young child or a child, or older than a child like a teenager, an adult, including elderly, suffering from a disease, notably from a muscle disorder.
90. The nutritional composition according to any of clauses 1 to 21, for use in increasing muscle force generation in a subject, wherein the subject is elderly, notably with sarcopenia and/or frailty.
91. The nutritional composition according to any of clauses 1 to 21, for use in promoting muscle health in a subject, wherein the subject is infant, a young child or a child.
92. The nutritional composition according to any of clauses 1 to 21, for use in promoting muscle health in a subject, wherein the subject is infant, a young child or a child born by C-section and/or preterm, and/or small for gestational age (SGA) and/or with a low, very low, or extremely low weight at birth (LWB, VLBW, or ELBW) and/or experienced intra-uterine growth retardation (IUGR).
93. The nutritional composition according to any of clauses 1 to 21, for use in promoting muscle health in a subject, wherein the subject is an infant, a young child or a child, or older than a child or a teenager, an adult, including elderly, recovering from a disease, notably from a muscle disorder.
94. The nutritional composition according to any of clauses 1 to 21, for use in promoting muscle health in a subject, wherein the subject is an infant, a young child or a child, or older than a child like a teenager, an adult, including elderly, suffering from a disease, notably from a muscle disorder.
95. The nutritional composition according to any of clauses 1 to 21, for use in promoting muscle health in a subject, wherein the subject is elderly, notably with sarcopenia and/or frailty.
96. The nutritional composition according to any of clauses 1 to 21, for use in promoting normal muscle function in a subject.
97. The nutritional composition according to any of clauses 1 to 21, for use in modulating muscle stem cells function in a subject.
98. The nutritional composition according to any of clauses 1 to 21, for use in promoting and/or increasing muscle stem cells function in a subject, wherein the subject is infant, a young child or a child.
99. The nutritional composition according to any of clauses 1 to 21, for use in promoting and/or increasing muscle stem cells function in a subject, wherein the subject is infant, a young child or a child born by C- section and/or preterm, and/or small for gestational age (SGA) and/or with a low, very low, or extremely low weight at birth (LWB, VLBW, or ELBW) and/or experienced intra-uterine growth retardation (IUGR).
100. The nutritional composition according to any of clauses 1 to 21, for use in promoting and/or increasing muscle stem cells function in a subject, wherein the subject is an infant, a young child or a child, or older than a child or a teenager, an adult, including elderly, recovering from a disease, notably from a muscle disorder.
101. The nutritional composition according to any of clauses 1 to 21, for use in promoting and/or increasing muscle stem cells function in a subject, wherein the subject is an infant, a young child or a child, or older than a child like a teenager, an adult, including elderly, suffering from a disease, notably from a muscle disorder.
102. The nutritional composition according to any of clauses 1 to 21, for use in promoting and/or increasing muscle stem cells function in a subject, wherein the subject is elderly, notably with sarcopenia and/or frailty.
103. The non-therapeutic use of the nutritional composition according to any of clauses 1 to 21, for improving and/or increasing and/or maintaining and/or stimulating and/or promoting and/or preventing at least one muscle condition in a subject, such as infant, a young child or a child, selected form the list consisting of:
   increasing muscle strength;
   increasing muscle force;
   increasing grip strength;
   improving endurance capacity;
   increasing muscle quality;
   increasing or maintaining muscle mass;
   increasing muscle specific force;
   increasing exercise capacity;
   increasing muscle force generation;
   promoting normal muscle function;
   modulating muscle stem cells function;
   promoting muscle stem cell function;
   increasing muscle stem cell function;
   and any combinations thereof.
104. The non-therapeutic use of the nutritional composition according to any of clauses 1 to 21, for improving and/or increasing and/or maintaining and/or stimulating and/or promoting and/or preventing at least one muscle condition in a subject, such as infant, a young child or a child, selected form the list consisting of:
   increasing muscle strength;
   increasing muscle force;
   increasing grip strength;
   and any combinations thereof.
105. The non-therapeutic use of the nutritional composition according to any of clauses 1 to 21, for improving at least one muscle condition in a subject, notably a healthy subject, like a healthy adult.
106. The non-therapeutic use of the nutritional composition according to any of clauses 1 to 21, for increasing at least one muscle condition in a subject, notably a healthy subject, like a healthy adult.
107. The non-therapeutic use of the nutritional composition according to any of clauses 1 to 21, for maintaining at least one muscle condition in a subject, notably a healthy subject, like a healthy adult.
108. The non-therapeutic use of the nutritional composition according to any of clauses 1 to 21, for stimulating at least one muscle condition in a subject, notably a healthy subject, like a healthy adult.
109. The non-therapeutic use of the nutritional composition according to any of clauses 1 to 21, for promoting at least one muscle condition in a subject, notably a healthy subject, like a healthy adult.
110. The non-therapeutic use of the nutritional composition according to any of clauses 1 to 21, for improving endurance capacity in a subject, notably a healthy subject, like a healthy adult.
111. The non-therapeutic use of the nutritional composition according to any of clauses 1 to 21, for maintaining muscle mass in a subject, notably a healthy subject, like a healthy adult.
112. The non-therapeutic use of the nutritional composition according to any of clauses 1 to 21, for increasing exercise capacity in a subject, notably a healthy subject, like a healthy adult.
113. The non-therapeutic use of the nutritional composition according to any of clauses 1 to 21, for modulating muscle health in a subject, notably a healthy subject, like a healthy adult.
114. The non-therapeutic use of the nutritional composition according to any of clauses 1 to 21, for promoting normal muscle function in a subject, notably a healthy subject, like a healthy adult.
115. The non-therapeutic use of the nutritional composition according to any of clauses 1 to 21, for modulating muscle stem cells function in a subject, notably a healthy subject, like a healthy adult.

## Claims

1. A nutritional composition comprising *L. reuteri* as a probiotic strain, wherein *L. reuteri* is pre-conditioned, wherein *L. reuteri* is preferably pre-conditioned with galacto-oligosaccharide (GOS),
the nutritional composition further comprising at least one further probiotic.

2. The nutritional composition according to claim 1, wherein the at least one further probiotic is selected form the list consisting of a *Dorea, a Bifidobacterium,* and any combination thereof.

3. The nutritional composition according to claim 1 or claim 2, wherein the at least one further probiotic is a *Bifidobacterium* selected form the list consisting of: *Bifidobacterium dentium, Bifidobacterium infantis,* and combination thereof.

4. The nutritional composition according to claim 1, wherein the at least one further probiotic is a *Limosilactobacillus.*

5. The nutritional composition according to claim 1 or claim 2, wherein the at least one further probiotic is *Dorea phocaeensis.*

6. The nutritional composition according to any of the preceding claims, wherein the pre-conditioned *L. reuteri* is obtained or obtainable by a process comprising the following steps:
a) cultivating a probiotic *L. reuteri* strain in the presence of galacto-oligosaccharide (GOS) in a growth medium, thereby pre-conditioning the probiotic *L. reuteri* strain;
b) harvesting the pre-conditioned *L. reuteri* from the growth medium, wherein the process is optionally comprising the following further step:
c) adding (fresh) GOS to the pre-conditioned probiotic *L. reuteri* strain obtained from step b).

7. The nutritional composition according to any of the preceding claims, further comprising at least one vitamin, wherein the at least one vitamin is optionally selected form the list consisting of: vitamin A, vitamin B1, vitamin B2, vitamin B3, vitamin B6, vitamin B12, vitamin C, vitamin D, vitamin E, vitamin K, vitamin B7, vitamin B5, vitamin B9, and any combinations thereof; preferably a combination comprising or consisting of high amounts of vitamin B3, vitamin C, vitamin D, vitamin E, vitamin K, vitamin B7, vitamin B5, vitamin B9, wherein the high amount are defined as being at least 30% more than in the draft of the Codex Standard For Follow-Up Formula For Older Infants And Product For Young Children CXS 156-1987 aged 6-36 months, report dated March 2023, appendix 2 from page 30, wherein the at least one vitamin is preferably present in the following amount:
vitamin A, in an amount ranging of from 1000 IU / 100 g to 3000 IU / 100 g, preferably of from 1500 IU / 100 g to 2600 IU / 100 g, more preferably of from 2000 IU / 100 g to 2500 IU / 100 g, wherein one IU for vitamin A is the biological equivalent of 0.3 microgram of retinol or of beta-carotene; and/or
vitamin B1, in an amount ranging of from 0.1 mg / 100 g to 1 mg / 100 g, preferably of from 0.3 mg / 100 g to 1 mg / 100 g, more preferably of from 0.5 mg / 100 g to 0.9 mg / 100 g;
and/or
vitamin B2, in an amount ranging of from 1 mg / 100 g to 3 mg / 100 g, preferably of from 1 mg / 100 g to 2 mg / 100 g, more preferably of from 1.1 mg / 100 g to 1.6 mg / 100 g; and/or vitamin B3, in an amount ranging of from 1.5 mg / 100 g to 12 mg / 100 g, preferably of from 3 mg / 100 g to 10 mg / 100 g, more preferably of from 4 mg / 100 g to 8 mg / 100 g; and/or vitamin B6, in an amount ranging of from 0 mg / 100 g to 1.3 mg / 100 g, preferably of from 0.2 mg / 100 g to 1 mg / 100 g, more preferably of from 0.3 mg / 100 g to 0.7 mg / 100 g;
and/or
vitamin B12, in an amount ranging of from 0 microgram / 100 g to 3 microgram / 100 g, preferably of from 1 microgram / 100 g to 2.5 microgram / 100 g, more preferably of from 1 microgram / 100 g to 2 microgram / 100 g; and/or
vitamin C, in an amount ranging of from 80 mg / 100 g to 400 mg / 100 g, preferably of from 85 mg / 100 g to 300 mg / 100 g, more preferably of from 100 mg / 100 g to 200 mg / 100 g;
and/or
vitamin D, in an amount ranging of from 130 IU / 100 g to 900 IU / 100 g, preferably of from 200 IU / 100 g to 700 IU / 100 g, more preferably of from 350 IU / 100 g to 500 IU / 100 g, wherein one IU for vitamin D is the biological equivalent of 0.025 microgram of cholecalciferol or ergocalciferol; and/or
vitamin E, in an amount ranging of from 11 IU / 100 g to 30 IU / 100 g, preferably of from 12 IU / 100 g to 25 IU / 100 g, more preferably of from 12 IU / 100 g to 20 IU / 100 g, wherein one IU for vitamin E is the biological equivalent of 0.67 mg of d-alpha-tocopherol, or of 0.9 mg of dl-alpha-tocopherol; and/or
vitamin K, in an amount ranging of from 23 microgram / 100 g to 100 microgram / 100 g, preferably of from 25 microgram / 100 g to 80 microgram / 100 g, more preferably of from 25 microgram / 100 g to 70 microgram / 100 g; and/or
vitamin B7, in an amount ranging of from 9 microgram / 100 g to 100 microgram / 100 g, preferably of from 12 microgram / 100 g to 60 microgram / 100 g, more preferably of from 15 microgram / 100 g to 30 microgram / 100 g; and/or
vitamin B5, in an amount ranging of from 2.1 mg / 100 g to 10 mg / 100 g, preferably of from 2.5 mg / 100 g to 8 mg / 100 g, more preferably of from 3 mg / 100 g to 6 mg / 100 g; and/or vitamin B9, in an amount ranging of from 51 microgram / 100 g to 350 microgram / 100 g, preferably of from 70 microgram / 100 g to 300 microgram / 100 g, more preferably of from 90 microgram / 100 g to 200 microgram / 100 g; and/or, wherein the at least one vitamin is preferably present in the following amount:
vitamin A, in an amount ranging of from 200 IU / 100 kcal to 1000 IU / 100 kcal, preferably of from 300 IU / 100 kcal to 800 IU / 100 kcal, more preferably of from 300 IU / 100 kcal to 600 IU / 100 kcal, wherein one IU for vitamin A is the biological equivalent of 0.3 microgram of retinol or of beta-carotene; and/or
vitamin B1, in an amount ranging of from 0.02 mg / 100 kcal to 0.5 mg / 100 kcal, preferably of from 0.05 mg / 100 kcal to 0.4 mg / 100 kcal, more preferably of from 0.05 mg / 100 kcal to 0.2 mg / 100 kcal; and/or
vitamin B2, in an amount ranging of from 0.02 mg / 100 kcal to 0.6 mg / 100 kcal, preferably of from 0.05 mg / 100 kcal to 0.5 mg / 100 kcal, more preferably of from 0.08 mg / 100 kcal to 0.5 mg / 100 kcal; and/or
vitamin B3, in an amount ranging of from 0.3 mg / 100 kcal to 3 mg / 100 kcal, preferably of from 0.6 mg / 100 kcal to 2.5 mg / 100 kcal, more preferably of from 0.8 mg / 100 kcal to 2 mg / 100 kcal; and/or
vitamin B6, in an amount ranging of from 0 mg / 100 kcal to 0.3 mg / 100 kcal, preferably of from 0.05 mg / 100 kcal to 0.25 mg / 100 kcal, more preferably of from 0.05 mg / 100 kcal to 0.20 mg / 100 kcal; and/or
vitamin B12, in an amount ranging of from 0 microgram / 100 kcal to 0.65 microgram / 100 kcal, preferably of from 0.05 microgram / 100 kcal to 0.6 microgram / 100 kcal, more preferably of from 0.05 microgram / 100 kcal to 0.5 microgram / 100 kcal; and/or vitamin C, in an amount ranging of from 17 mg / 100 kcal to 100 mg / 100 kcal, preferably of from 20 mg / 100 kcal to 80 mg / 100 kcal, more preferably of from 20 mg / 100 kcal to 50 mg / 100 kcal; and/or
vitamin D, in an amount ranging of from 26 IU / 100 kcal to 150 IU / 100 kcal, preferably of from 40 IU / 100 kcal to 120 IU / 100 kcal, more preferably of from 50 IU / 100 kcal to 130 IU / 100 kcal, wherein one IU for vitamin D is the biological equivalent of 0.025 microgram of cholecalciferol or ergocalciferol; and/or
vitamin E, in an amount ranging of from 2.0 IU / 100 kcal to 12 IU / 100 kcal, preferably of from 2.5 IU / 100 kcal to 10 IU / 100 kcal, more preferably of from 2.6 IU / 100 kcal to 5 IU / 100 kcal, wherein one IU for vitamin E is the biological equivalent of 0.67 mg of d-alpha-tocopherol, or of 0.9 mg of dl-alpha-tocopherol; and/or
vitamin K, in an amount ranging of from 4.6 microgram / 100 kcal to 25 microgram / 100 kcal, preferably of from 5 microgram / 100 kcal to 20 microgram / 100 kcal, more preferably of from 5 microgram / 100 kcal to 15 microgram / 100 kcal; and/or
vitamin B7, in an amount ranging of from 2 microgram / 100 kcal to 15 microgram / 100 kcal, preferably of from 2.5 microgram / 100 kcal to 12 microgram / 100 kcal, more preferably of from 3 microgram / 100 kcal to 10 microgram / 100 kcal; and/or
vitamin B5, in an amount ranging of from 0.43 mg / 100 kcal to 2 mg / 100 kcal, preferably of from 0.5 mg / 100 kcal to 1.6 mg / 100 kcal, more preferably of from 0.55 mg / 100 kcal to 1.2 mg / 100 kcal; and/or
vitamin B9, in an amount ranging of from 13 microgram / 100 kcal to 80 microgram / 100 kcal, preferably of from 15 microgram / 100 kcal to 60 microgram / 100 kcal, more preferably of from 20 microgram / 100 kcal to 45 microgram / 100 kcal.

8. The nutritional composition according claim 7, comprising high amounts of the following at least one vitamin:
• vitamin B3;
• vitamin D;
• vitamin B9;
• vitamin B3, vitamin D and vitamin B9;
• vitamin B3 and vitamin D;
• vitamin B3 and vitamin B9;
• vitamin D and vitamin B9;
• vitamin B3 and vitamin B7;
• vitamin D and vitamin B7;
• vitamin B9 and vitamin B7;
• vitamin B3, vitamin D and vitamin B7;
• vitamin B3, vitamin D, vitamin B9 and vitamin B7;
• vitamin B3, vitamin D and vitamin K;
• vitamin B3, vitamin D, vitamin B7 and vitamin K;
• vitamin B3, vitamin D, vitamin B7, vitamin B9 and vitamin K;
• vitamin B3, vitamin D, vitamin B7, vitamin B9, vitamin C and vitamin K;
• vitamin B3, vitamin D, vitamin B7, vitamin B9, vitamin C, vitamin E and vitamin K;
• vitamin D and vitamin B5;
• vitamin B9 and vitamin B5;
• vitamin B3, vitamin D, vitamin B5 and vitamin B9;
• vitamin B3, vitamin D and vitamin B5;
• vitamin B3, vitamin B9 and vitamin B5;
• vitamin D, vitamin B9 and vitamin B5;
• vitamin B3, vitamin D, vitamin B7, vitamin B9, vitamin C, vitamin **E,** vitamin K and vitamin B5;
preferably comprising high amounts of the following at least one vitamin:
• vitamin D;
• vitamin B3, vitamin D and vitamin B9;
• vitamin B3 and vitamin D;
• vitamin D and vitamin B9;
• vitamin D and vitamin B7;
• vitamin B3, vitamin D and vitamin B7;
• vitamin B3, vitamin D, vitamin B9 and vitamin B7;
• vitamin B3, vitamin D and vitamin K;
• vitamin B3, vitamin D, vitamin B7 and vitamin K.

9. The nutritional composition according to any of the preceding claims, further comprising lipids comprising fatty acid (FA) triacylglycerols (TAG, said fatty acid TAG comprising preferably stearic, palmitic and myristic esters, wherein the sum of the amount of stearic, palmitic and myristic esters at the sn-1(3) position of the TAG is less than 13.0 wt-% of TAG and the myristic acid represents up to 3.7 wt-% of the total FA, the palmitic acid represents up to 11.1 wt-% of the total FA and stearic acid represents up to 4.6 wt-% of the total FA, wherein the short chain fatty acid (SCFA) are present in preferably less than 40 wt-%, wherein the mono-unsaturated fatty acids (MUFA) are present in preferably more than 35.1 wt-%, wherein the lipids preferably comprise a mixture of oils selected from:
16.9 wt-% to 22.0 wt-% of rapeseed oil, 11.5 to 14.5 wt-% of sunflower oil, 40.0 to 58 wt-% of high oleic sunflower oil, and 15.0 wt-% to 20.5 wt-% of coconut oil.

10. The nutritional composition according to any of the preceding claims, further comprising at least one mineral, wherein the at least one mineral is preferably selected form the list consisting of: calcium, phosphorous, magnesium, iron, zinc, potassium, copper, manganese, selenium, sodium and any combinations thereof; preferably selenium, manganese, a combination comprising or consisting of selenium, manganese, or a combination comprising or consisting of magnesium, manganese, selenium, wherein the at least one mineral is preferably present in the following amount:
magnesium, in an amount ranging of from 30 mg / 100 g to 200 mg / 100 g, preferably of from 40 mg / 100 g to 150 mg / 100 g, more preferably of from 15 mg / 100 g to 150 mg / 100 g;
and/or
manganese, in an amount ranging of from 70 microgram / 100 g to 800 microgram / 100 g, preferably of from 100 microgram / 100 g to 450 microgram / 100 g, more preferably of from 100 microgram / 100 g to 250 microgram / 100 g; and/or
selenium, in an amount ranging of from 7 microgram / 100 g to 120 microgram / 100 g, preferably of from 10 microgram / 100 g to 50 microgram / 100 g, more preferably of from 10 microgram / 100 g to 25 microgram / 100 g;
and/or
magnesium in an amount ranging of from 6 mg / 100 kcal to 30 mg / 100 kcal, preferably of from 8 mg / 100 kcal to 25 mg / 100 kcal, more preferably of from 10 mg / 100 kcal to 15 mg / 100 kcal; and/or
manganese in an amount ranging of from 15 microgram / 100 kcal to 250 microgram / 100 kcal, preferably of from 20 microgram / 100 kcal to 200 microgram / 100 kcal, more preferably of from 20 microgram / 100 kcal to 70 microgram / 100 kcal; and/or
selenium in an amount ranging of from 2.5 microgram / 100 kcal to 12 microgram / 100 kcal, preferably of from 2.8 microgram / 100 kcal to 9 microgram / 100 kcal, more preferably of from 3.0 microgram / 100 kcal to 9 microgram / 100 kcal.

11. The nutritional composition according to any of the preceding claims, wherein the nutritional composition is an infant formula, a starter infant formula, a follow-on or follow-up infant formula, a baby food, an infant cereal composition, a growing-up milk, a fortifier or a supplement.

12. The nutritional composition according to any of the preceding claims, for use in improving and/or increasing and/or maintaining and/or treating and/or stimulating and/or promoting and/or preventing at least one muscle condition in a subject, wherein the subject is preferably an infant, a young child, or a child, wherein the subject was optionally born by C- section and/or preterm, and/or small for gestational age (SGA) and/or with a low, very low, or extremely low weight at birth (LWB, VLBW, or ELBW) and/or experienced intra-uterine growth retardation (IUGR).

13. The nutritional composition for use according to claim 11 or claim 12, for use in improving and/or increasing and/or maintaining and/or treating and/or stimulating and/or promoting and/or preventing at least one muscle condition in a subject, selected form the list consisting of:
increasing muscle strength;
increasing muscle force;
increasing grip strength;
improving endurance capacity;
increasing muscle quality;
increasing or maintaining muscle mass;
increasing muscle specific force;
increasing exercise capacity;
modulating muscle health;
increasing muscle force generation;
treating a muscle disorder;
preventing a muscle disorder;
(promoting muscle health);
promoting normal muscle function;
modulating muscle stem cells function;
promoting muscle stem cell function;
increasing muscle stem cell function;
and any combinations thereof;
wherein the nutritional composition is preferably for use in increasing muscle strength and/or increasing muscle force and/or increasing grip strength and/or increasing or maintaining muscle mass in a subject, or
wherein the nutritional composition is preferably for use in treating a muscle disorder in a subject, such as infant, a young child or a child.

14. Use of a nutritional composition according to any of claims 1 to 11, for improving and/or increasing and/or maintaining and/or treating and/or stimulating and/or promoting at least one muscle condition in a subject.

15. The nutritional composition according to any of claims 1 to 11, for use in :
improving and/or increasing at least one muscle condition in a subject, wherein the subject is an infant, a young child or a child, or older than a child or a teenager, an adult, including elderly, recovering from a disease, notably from a muscle disorder; or
increasing muscle strength in a subject, wherein the subject is an infant, a young child or a child, or older than a child like a teenager, an adult, including elderly, suffering from a disease,
notably from a muscle disorder; or , for use in increasing muscle quality in a subject, wherein the subject is an infant, a young child or a child, or older than a child like a teenager, an adult, including elderly, suffering from a disease, notably from a muscle disorder; or
increasing muscle specific force in a subject, wherein the subject is an infant, a young child or a child, or older than a child or a teenager, an adult, including elderly, recovering or suffering from a disease, notably from a muscle disorder; or for use in increasing muscle force generation in a subject, wherein the subject is an infant, a young child or a child, or older than a child or a teenager, an adult, including elderly, recovering or suffering from a disease,
notably from a muscle disorder; or
promoting muscle health in a subject, wherein the subject is an infant, a young child or a child, or older than a child or a teenager, an adult, including elderly, recovering or suffering from a disease, notably from a muscle disorder; or
promoting and/or increasing muscle stem cells function in a subject, wherein the subject is an infant, a young child or a child, or older than a child or a teenager, an adult, including elderly, recovering from a disease, notably from a muscle disorder.
